# EUROPÄISCHE PATENTANMELDUNG

(11) **EP 2 273 270 A1**
(43) Veröffentlichungstag der Anmeldung: **12.01.2011**
(21) Anmeldenummer: 10176218.5
(22) Anmeldetag: 27.08.1999
(51) Int. Cl.: G01N 33/551, B01J 19/00, B01L 3/00, C12N 15/10, C12P 19/34, G01N 21/25

(54) **Verfahren und Vorrichtung zur Herstellung und/oder Analyse von biochemischen Reaktionsträgern**

(30) Priorität: 28.08.1998 DE 19839254; 28.08.1998 DE 19839255; 28.08.1998 DE 19839256; 19.02.1999 DE 19907080; 27.05.1999 DE 19924327
(62) Teilanmeldung aus: 99968261.0
(71) Anmelder: febit holding GmbH, 69120 Heidelberg (DE)
(72) Erfinder: Lindner, Hans, 70569 Stuttgart (DE); Müller, Manfred, 80689 München (DE); Stähler, Cord, 69493 Hirschberg a.d. Bergstrasse/Großsachsen (DE); Stähler, Fritz, 69469 Weinheim (DE); Stähler, Peer, 68167 Mannheim (DE)
(74) Vertreter: Tiesmeyer, Johannes

(57) **Zusammenfassung**

Die Erfindung betrifft ein Verfahren und eine Vorrichtung zur Herstellung eines mit biologisch oder chemisch funktionellen Materialien beschichteten Trägers (BioChip), umfassend
eine Belichtungsmatrix (10), die zur Erzeugung eines wahlweise einstellbaren Belichtungsmusters mittels einer programmierbaren Steuereinrichtung (12) steuerbar ist,
einen die Belichtungsmatrix (10) tragenden Rahmen mit einer Trägerhalterung zur Aufnahme und Positionierung eines betreffenden Trägers (4) relativ zur Belichtungsmatrix, so dass von der Belichtungsmatrix (10) erzeugte Belichtungsmuster auf die Oberfläche des Trägers (4) projizierbar sind, um diesen zur Aktivierung photoaktivierbarer Gruppen auf mindestens einem vorbestimmten Bereich seiner Oberfläche ortsspezifisch zu belichten,
**dadurch gekennzeichnet, dass** die Belichtungsmatrix als selbstemittierende Belichtungsmatrix, insbesondere als Diodenarray oder/und Laserarray, ausgebildet ist. Vorzugsweise ist auch eine Lichtdetektionseinrichtung (6) mit einer Lichtsensormatrix (16) zur Beobachtung des Trägers (4) vorgesehen, mittels welcher der Träger (4) automatisch zu erkennen und/oder zu kalibrieren ist.

## Beschreibung

Die Erfindung befaßt sich mit der Verwendung einer zur Erzeugung eines wahlweise einstellbaren Belichtungsmusters steuerbaren Belichtungsmatrix, insbesondere programmierbaren Lichtquellenmatrix, im Bereich der Biotechnologie im Allgemeinen und für die Herstellung, Manipulation und Analyse von opto-fluidischen Reaktionsträgern im Speziellen.

Durch eine Miniaturisierung bei gleichzeitiger Funktionsintegration von Bauteilen, Komponenten und ganzen Systemen werden in vielen Technologiefeldern neue Anwendungen erschlossen. Diese Anwendungen reichen von der Sensorik über Mikrosystemtechnik (z.B. komplexe BioChips unter Verwendung der Halbleitertechnik) bis zur Aktorik (z.B. in Form von Mikropumpen). Die Branchen reichen vom klassischen Maschinenbau über Automobil- und Luftfahrtindustrie bis zur Medizintechnik und der zunkunftsweisenden Biotechnologie. In der Medizintechnik werden beispielsweise neue Implantate entwickelt und im Bereich der Pharmaindustrie werden neue Technologien für die effiziente Entwicklung neuer Medikamente und Diagnosesysteme mit enormen Aufwand vorangetrieben. Von dieser Entwicklung profitiert aufgrund des großen Potentials besonders die Biotechnologie.

Für eine wirtschaftliche Produktion im Mikrobereich werden neue Verfahren entwickelt, die den veränderten Randbedingungen gerecht werden. Das gleiche gilt für die benötigten Inspektionstechniken für die Überwachung der miniaturisierten Vorgänge.

Für die Grundlagenforschung in den Biowissenschaften und für die medizinische Diagnostik sowie einige andere Disziplinen ist die Erfassung biologisch relevanter Information (meist in Form genetischer Information) in definiertem Untersuchungsmaterial von herausragender Bedeutung. Dabei liegt die genetische Information in Form einer enormen Vielfalt von unterschiedlichen Nukleinsäuresequenzen vor, der DNA (desoxyribonucleic acid). Die Realisation dieser Information führt über die Herstellung von Abschriften der DNA in RNA (ribonucleic acid) meist zur Synthese von Proteinen, die ihrerseits häufig an biochemischen Reaktionen beteiligt sind.

Ein leistungsfähiges System-Format für die Erfassung dieser Fülle an Informationen ist der sog. BioChip. Unter BioChips werden in diesem Zusammenhang stark miniaturisierte, hoch parallele Assays verstanden. Die Detektion von bestimmten Nukleinsäuren und die Bestimmung der Abfolge der vier Basen in der Kette der Nukleotide (Sequenzierung) liefert wertvolle Daten für Forschung und angewandte Medizin. In der Medizin konnte in stark zunehmendem Maße durch die in vitro-Diagnostik (IVD) ein Instrumentarium zur Bestimmung wichtiger Patientenparameter entwickelt und dem behandelnden Arzt zur Verfügung gestellt werden. Für viele Erkrankungen wäre eine Diagnose zu einem ausreichend frühen Zeitpunkt ohne dieses Instrumentarium nicht möglich. Hier hat sich die genetische Analyse als wichtiges neues Verfahren etabliert (z.B. Falldiagnose von Infektionskrankheiten wie HIV oder HBV, genetische Prädisposition für bestimmte Krebsarten oder andere Erkrankungen, oder in der Forensik). In enger Verzahnung von Grundlagenforschung und klinischer Forschung konnten die molekularen Ursachen und (pathologischen) Zusammenhänge einiger Krankheitsbilder bis auf die Ebene der genetischen Informationen aufgeklärt werden. Diese Entwicklung steht allerdings noch am Anfang, und gerade für die Umsetzung in Therapiestrategien bedarf es stark intensivierter Anstrengungen. Insgesamt haben die Genomwissenschaften und die damit verbundene Nukleinsäureanalytik sowohl zum Verständnis der molekularen Grundlagen des Lebens als auch zur Aufklärung sehr komplexer Krankheitsbilder und pathologischer Vorgänge wichtige Beiträge geleistet. Darüber hinaus liefert die genetische bzw. gentechnische Analyse bereis heute ein breites diagnostisches Methodenspektrum.

Die weitere Entwicklung in der medizinischen Versorgung wird durch die Explosion der Kosten belastet, die mit entsprechend aufwendigen Verfahren verbunden sind. So kostet die Bestimmung von genetischen Risikofaktoren durch Sequenzierung derzeit noch mehrere hundert bis mehrere tausend US-Dollar. Hier muß nicht nur auf die Realisation der Möglichkeiten an diagnostischem und therapeutischem Nutzen gedrängt, sondern auch eine Integration in ein tragfähiges, finanzierbares Gesundheitssystem vorangetrieben werden.

Eine Anwendung entsprechender Technologien in der Forschung kann ebenfalls nur dann in breitem Umfang und auch im akademischen Bereich erfolgen, wenn die damit verbundenen Kosten reduziert werden. Hier zeichnet sich ein Paradigmenwechsel für die Forschung in den Biowissenschaften ab:
Das Nadelöhr der Entschlüsselung primärer genetischer Information (Basensequenz im Genom) und der Erfassung des genetischen Aktivitätszustandes (als Boten-RNA umgeschriebene Gene) von Zellen und Geweben fällt mit der Verfügbarkeit ausreichend billiger, leistungsfähiger und flexibler Systeme weg. Die Arbeit kann sich dann auf die (sehr komplexe) Aufgabe der Auswertung und Kombination der betreffenden Daten konzentrieren. Daraus sollten sich neue Erkenntnishorizonte für die Biologie und in der Folge neue biomedizinische Therapien und Diagnosemöglichkeiten ergeben.

Bei den vorstehend bereits genannten BioChips handelt es sich um miniaturisierte hybride Funktionselemente mit biologischen und technischen Komponenten, z.B. auf der Oberfläche eines Trägers (Außenoberfläche oder/und Innenoberfläche) immobilisierte Biomoleküle, die als spezifische Interaktionspartner dienen können, und eine Matrix, z.B. Silizium-Matrix. Häufig weist die Struktur dieser Funktionselemente Reihen und Spalten auf; man spricht dann von Chip-"Arrays". Da tausende von biologischen bzw. biochemischen Funktionselementen auf einem solchen Chip angeordnet sein können, müssen diese in der Regel mit mikrotechnischen Methoden angefertigt werden.

Als Verfahren für die Herstellung dieser Arrays kommen im Wesentlichen zwei Prinzipien zur Anwendung. Das Aufbringen fertiger Sonden bzw. Funktionselementen auf den Reaktionsträger, was derzeit überwiegend angewendet wird, oder das In-situ-Synthetisieren der Sonden auf dem Träger. Als Geräte werden sogenannte mikrofluidische Spotter für beide Prinzipien angewandt. Für die In-situ-Synthese kommen auch photolithographische Verfahren zur Anwendung.

Als biologische und biochemische Funktionselemente kommen insbesondere in Frage: DNA, RNA, PNA, (bei Nukleinsäuren und ihren chemischen Derivaten können z.B. Einzelstränge, Triplex-Strukturen oder Kombinationen hiervon vorliegen), Saccharide, Peptide, Proteine (z.B. Antikörper, Antigene, Rezeptoren), Derivate der kombinatorischen Chemie (z.B. organische Moleküle), Zellbestandteile (z.B. Organellen), Zellen, Mehrzeller, Zellverbände.

Für die Anwendungen in der Halbleitertechnologie gibt es am Markt verfügbar eine Vielzahl von photolithographischen Systemen zur belichtungsabhängigen Erzeugung feiner und feinster Strukturen mit Licht unterschiedlicher Wellenlänge (Energie) bis unter 200 nm. Je feiner die zu erzeugenden Strukturen sind, desto kürzer muß auch die verwendete Wellenlänge sein. So können Strukturen im sub-µm-Bereich, welche an sich schon im Bereich der Wellenlänge des sichtbaren Lichtes (400-800 nm) liegen, nur mit hochenergetischer Strahlung deutlich kürzerer Wellenlänge erzeugt werden.

Photolithographiesysteme bestehen prinzipiell aus einer Lampe als Energie- bzw. Lichtquelle und einer photolithographischen Maske, welche durchsichtige und undurchsichtige Bereiche aufweist und so im Durchlicht-Strahlengang ein Belichtungsmuster erzeugt. Dieses Belichtungsmuster wird durch optische Elemente auf dem zu belichtenden Gegenstand abgebildet (z.B. um den Faktor 100 verkleinert). Dadurch wird eine Linie auf der Maske von 0,1 mm Breite auf 10 µm reduziert. Üblicherweise werden für die Herstellung einer Mikrostruktur in bzw. auf einem Silicium-Wafer 10 bis 30 Beüchtungsschritte benötigt. Auf diese Anzahl sind die Systeme ausgelegt und ermöglichen mittels Magazinen und Handhabungsgeräten einen automatischen Maskenwechsel.

Aus einer quasi-makroskopischen Struktur der Maske wird damit eine mikrostrukturierte Abbildung auf dem zu belichtenden Körper, z.B. dem Silicium-Wafer. Zur Erzeugung einer photolithographischen Maske werden ebenfalls wieder photolithographische Systeme eingesetzt, welche natürlich nur eine entsprechend geringere Auflösung und je nach Herstellverfahren auch nur einen entsprechend niedrigeren Energieeintrag benötigen. Es handelt sich dabei um einen zyklischen Vorgang, welcher durch das große Marktvolumen der Halbleiterindustrie sehr weit vorangetrieben und perfektioniert wurde.

Für die Herstellung der Photolithographie-Masken kommen bei der Firma GeSim bereits LCD-Photoplotter der Firma Mivatec zum Einsatz. Dies ist möglich, da die Masken-Strukturen von der Größe der Struktur her sowie der benötigten Wellenlänge her eine Belichtung im Bereich des sichtbaren Lichtes erlauben. Damit ist eine schnellere und flexiblere Herstellung von Masken möglich. Dies ist für die Halbleitertechnologie aufgrund der begrenzten Anzahl an benötigten Masken ausreichend, da erst der Funktionstest den Erfolg der Mikrostrukturierung zeigt und somit in der Regel immer ausreichend Zeit für die Produktion neuer oder verbesserter Masken bleibt. Insgesamt ist die Herstellung der Masken jedoch teuer, zeitaufwendig und wenig flexibel.

Bei der Verwendung der Photolithographhie für die lichtinduzierte in situ-Synthese von DNA (Synthese direkt auf dem BioChip) werden vom Institut Affymax sowie von der Firma Affymetrix bereits handelsübliche Belichtungssysteme zur Herstellung von hochdichten DNA-Mikroarrays eingesetzt (Referenzen: US 5,744,305, US 5,527,681, US 5,143,854, US 5,593,839, US 5,405,783). Die eingesetzte Wellenlänge ist auf 300-400 nm beschränkt. Für jede Änderung des Belichtungsmusters ist ein Wechsel der Maske erforderlich. Dies ist extrem hinderlich, da für die Produktion zum Beispiel eines DNA-Arrays mit 25 Bausteine langen Oligonukleotiden (25mere) je Meßplatz ca. 100 individuelle Belichtungszyklen benötigt werden.

Im Allgemeinen haben die Reaktionsträger eine 2D-Basisfläche für das Beschichten mit biologisch oder biochemisch funktionellen Materialien. Die Basisflächen können beispielweise auch von Wänden einer oder mehrerer Kapillaren oder von Kanälen gebildet sein. Eine Weiterführung der Geometrie ist eine 3D-Struktur, bei der die Analyse und gegebenenfalls auch Manipulation respektive Steuerung von Reaktionen in einer 2D-Anordnung erfolgen.

Vor allem in den USA wird die Entwicklung von miniaturisierten BioChips mit enormen Mitteln vorangetrieben.

Zum Stand der Technik kann z.B. auf folgende Publikationen hingewiesen werden:
1. Nature Genetics, Vol. 21, supplement (gesamt), Jan. 1999 (BioChips)
2. Nature Biotechnology, Vol. 16, S. 981-983, Okt. 1998 (BioChips)
3. Trends in Biotechnology, Vol. 16, S. 301-306, Jul. 1998 (BioChips).

Wichtige Anwendungsfelder für miniaturisierte, parallele Assays und damit die Anwendung der vorliegenden Erfindung sind:
Molekulare Diagnostik (mit in vitro-Diagnostik, klinischer Diagnostik, genetischer Diagnostik)/Pharmaka-Entwicklung (Substanzentwicklung, Austesten, Screening etc.)/biologischeGrundlagenforschung (u.a. Genomik, Transkriptom, Proteom, Physiom)/molekulare Interaktionen/Analyse und Screening nach Pathogenen (Viroide, Prionen, Viren, Prokaryonten, Eukaryonten)/Onkologie/Umweltmonitoring/Lebensmittelanalytik/Forensik/ Screening von medizinischen Produkten (u.a. Produkte aus Blut)/Detektion, Analyse und Screening von Transgenen (Pflanzen, Tiere, Bakterien, Viren, Züchtung, Freilandversuche)/Cytologie (u.a. Zellassys)/Histologie/alle Formen von Nukleinsäureanalysen (u.a. Sequenzanalyse, Kartierung, Expressionsprofile)/SNPs/Pharmakogenomik/funktionelle Genomik.

Der Erfindung liegt die Aufgabe zugrunde, ein Verfahren und eine Vorrichtung anzugeben, welche eine flexiblere und schnellere Herstellung und eine effizientere Analyse von miniaturisierten, hochparallelen Reaktionsträgern ermöglicht.

Verfahren und Vorrichtung sollten zudem die Integration von Herstellung und Analyse in ein Gerät ermöglichen. Weiterhin ist die Schaffung einer Basis für eine vollständige Automatisierung aller Vorgänge bei Herstellung und Analyse beabsichtigt.

Das erfindungsgemäße Verfahren zur Herstellung eines mit biologisch oder biochemisch funktionellen Materialien beschichteten Reaktionsträgers umfaßt folgende Schritte:
(a) Bereitstellen eines Trägers mit einer Oberfläche, die photoaktivierbare Gruppen aufweist,
(b) Aktivieren der photoaktivierbaren Gruppe auf mindestens einem vorbestimmten Bereich der Trägeroberfläche durch ortsspezifische Belichtung des Trägers mit einer Belichtungsmatrix, die zur Erzeugung eines wahlweise einstellbaren Belichtungsmusters steuerbar ist,
(c) ortsspezifisches Binden von biologisch oder chemisch funktionellen Materialien oder Bausteinen für solche Materialien auf mindestens einem der vorbestimmten Bereiche und
(d) gegebenenfalls Wiederholen der Aktivierungs- und Bindeschritte auf gleichen oder/und unterschiedlichen vorbestimmten Bereichen.

Der Träger ist eine mit biochemischen oder biologischen Materialien bzw. Rezeptoren oder Bausteinen davon bestückbare oder bestückte Festphase. Der Träger kann eine planare Oberfläche oder eine mit Vertiefungen, z.B. Kanälen versehene Oberfläche aufweisen. Die Kanäle sind vorzugsweise Mikrokanäle mit einem Querschnitt von z.B. 10 - 1000 µm. Die Kanäle können - abhängig von den Oberflächeneigenschaften - Kapillarkanäle, aber auch Kanäle ohne Kapillarwirkung (z.B. aufgrund von Beschichtung mit Teflon) sein. Der Träger ist zumindest teilweise im Bereich der zu bestückenden Reaktionsbereiche optisch transparent.

Die Verwendung einer Belichtungsmatrix, die zu einer Erzeugung eines wahlweise einstellbaren Belichtungsmusters steuerbar ist, ermöglicht eine große Flexibilität bei der Herstellung oder/und Manipulation oder/und Analyse von opto-fluidischen Reaktionsträgern und insbesondere eine schnellere Präparation von Reaktionsträgern als dies bisher möglich war. Im Gegensatz zu der Erzeugung von entsprechend feinauflösenden Belichtungsmustern in einer Photolithographiemaschine mittels invarianter individueller Masken, die bei einem Wechsel des Belichtungsmusters gewechselt werden müssen, kann mit einer steuerbaren Belichtungsmatrix jedes prinzipiell mögliche Belichtungsmuster durch einfache Ansteuerung der Belichtungsmatrix von einem Steuerrechner aus erzeugt und geändert werden. In einem Herstellungsprozess können damit an einem Tag prinzipiell hunderte bis tausende unterschiedliche Reaktionsträger mit einer Vielzahl an individuellen Reaktionsbereichen erzeugtund analysiert werden, was bislang nicht möglich war.

Die vorbestimmten Reaktionsbereiche, an denen eine ortsspezifische Belichtung des Trägers durchgeführt werden soll, werden für eine aktuelle Anwendung vorzugsweise automatisch durch ein Programm ausgewählt, mit dem eine Steuerung und Zuordnung der Reaktionsbereiche zu einem oder mehreren Reaktionsträgern nach den Kriterien Syntheseeffizienz, optimale Synthesebedingungen, z.B. Temperatur etc., optimale Analysebedingungen, z.B. Hybridisierungstemperatur unter Berücksichtigung benachbarter Bereiche, ermöglicht wird. Nach Herstellung des Trägers kann gegebenenfalls ein Wechsel des Trägers und eine Fortsetzung des Verfahrens ab Schritt (a) vorgesehen sein. Dabei kann Schritt (c), das ortsspezifische Binden von biologisch oder chemisch funktionellen Materialien oder Bausteinen für solche Materialien ebenso wie im vorhergehenden Zyklus oder aber unter Berücksichtigung der Informationen aus einem vorhergehenden Synthesezyklus umfassen.

Durch die Programmierbarkeit bzw. elektronische Steuerbarkeit der Belichtungsmatrix entfällt der Austausch sowie die Erzeugung der Maskeneinheiten, wie sie bei den photolithographischen Methoden erforderlich waren. Die Belichtungsmustererzeugung ist somit nicht mehr mit einem Aufwand für die Herstellung, das Auswechseln, Positionieren, Lagern und Optimieren von Belichtungsmasken verbunden. Damit wird insbesondere die in situ-Synthese von Reaktionsträgern (z.B. DNA-Mikro-Arrays) für einen breiten Einsatz zugänglich. Gemäß einer bevorzugten Ausführungsform der Erfindung verwendet man eine Belichtungsmatrix, die mit einer Auflösung von mindestens 500 Punkten pro cm² belichten kann.

Die Belichtungsmatrix und die zugeordnete Lichtquelle dienen grundsätzlich dazu, das gewünschte Belichtungsmuster für die Steuerung/Anregung photochemischer Prozesse oder ggf. für die Analyse einer Reaktionsträger-Matrix bereitzustellen. Dabei kann gemäß einer Variante die Lichtintensität und/oder die Wellenlänge je Lichtpunkt der Belichtungsmatrix bzw. des Belichtungsmusters auf dem Reaktionsträger wahlweise moduliert werden.

Vorzugsweise wird als Belichtungsmatrix eine steuerbare Reflexionsmatrix herangezogen, welche Licht ortsselektiv nach Maßgabe ihrer Ansteuerung in eine bestimmte Richtung (hier Richtung des Reaktionsträgers) reflektiert. Solche reflektierenden Flächenlichtmodulatoren mit gesteuert deformierbaren Spiegelanordnungen zur Erzeugung von Lichtmustern können insbesondere als Lichtmodulatoren mit viskoelastischen Steuerschichten oder als Lichtmodulatoren mit mikromechanischen Spiegelarrays realisiert sein. Zu der Technologie solcher Lichtmodulatoren mit viskoelastischen Steuerschichten und Lichtmodulatoren mit mikromechanischen Spiegelarrays wird auf betreffende Datenblätter des Fraunhofer-Instituts für mikroelektronische Schaltungen und Systeme verwiesen, die dieser Anmeldung als Anlage beigefügt sind. Der Vorzug solcher steuerbarer Reflexionsmatrizen liegt insbesondere darin, daß sie für einen weiten Spektralbereich des Lichtes vom UV bis IR verfügbar sind, beispielsweise in einem Wellenlängenbereich von 200-2000 nm. Insbesondere für die Übertragung von energiereicher Strahlung im UV-Bereich sowie allgemein bei hohen Energiedichten pro Fläche sind neueste Entwicklung von steuerbaren Reflexionsmatrizen in 40V-CMOS Technik vorteilhaft. Durch die Betriebsspannung von 40 V sind die Matrizen entsprechend unempfindlich. Ein weiterer Vorteil liegt darin, daß eine derartige Reflexionsmatrix bei entsprechender Beleuchtung mit einem über die Matrixfläche ausgedehnten Lichtfeld eine zeitlich parallele Belichtung aller zu belichtenden Stellen in dem Belichtungsmuster ermöglicht. Diese Möglichkeit der Parallelität der Belichtung eines Reaktionsträgers hat Auswirkungen auf die Herstellungsdauer (bei in situ-Synthesen) auf die Möglichkeiten zur Online-Kontrolle und Auswertung (keine Artefakte durch Zeitspannen zwischen Meßpunkten etc.) und auf die Möglichkeiten der Manipulation, z.B. bei Zell-Arrays oder anderen biologischen Komponenten eines Reaktionsträgers (etwa bei Retina-Präparaten oder lichtabhängiger neuronaler Aktivität).

Sofern es auf Parallelität der Belichtung nicht sehr streng ankommt, kann anstelle der ganzflächigen Bestrahlung der Belichtungsmatrix eine Rasterung bzw. Abtastung der Belichtungsmatrix mit einem gebündelten Strahl, z.B. Laserstrahl, erfolgen, um auf dem bzw. in dem Reaktionsträger das gewünschte Lichtmuster nach Maßgabe der Ansteuerung der Belichtungsmatrix zu erzeugen. Es können somit verschiedenste Lichtquellen Verwendung finden, so z.B. auch Lichtquellen, deren Emissionsspektrum oder Emissionswellenlänge wahlweise änderbar ist, z.B. ein N²-Laser, so daß z.B. eine Anregung mehrerer signalgebender Fluoreszenzstoffe auf oder in dem Reaktionsträger mit unterschiedlichen Wellenlängen möglich ist (dies ist eine Art 2D-Spektroskopie).

Eine weitere Klasse möglicher Belichtungsmatrizen für die Verwendung gemäß der vorliegenden Erfindung stellen die Lichtquellen-Arrays, d.h. matrixförmige Anordnungen kleinster Lichtquellen dar, die individuell ansteuerbar sind. Hierbei kann es sich z.B. um Mikrolaser-Arrays, Mikrodioden-Arrays oder dergleichen handeln. Mittlerweile sind UV-Leuchtdioden verfügbar, deren Emissionswellenlänge bei 370 nm liegt. Derartige UV-Leuchtdioden werden unter der Typenbezeichnung NSHU 590 und NSHU 550 von der Roithner Lasertechnik, A-1040 Wien, Fleischmanngasse 9, vertrieben. Die entsprechende UV-Leuchtdioden-Technik kann zur Realisierung eines Dioden-Arrays, insbesondere Mikrodioden-Arrays, herangezogen werden.

Die einzelnen ansteuerbaren Punkte eines solchen Lichtquellen-Arrays (Lichtquellenmatrix) entsprechen demnach den einzelnen Beleuchtungspunkten auf dem Reaktionsträger in den einzelnen Reaktionsbereichen, wobei das erzeugte Belichtungsmuster bedarfsweise mit Hilfe geeigneter optischer Baukomponenten verkleinert werden kann. Eine solche (selbstleuchtende) Lichtquellenmatrix unterscheidet sich von Belichtungsmatrizen, die als "Lichtventil" arbeiten, wie z.B. LCD's, und solchen, die als Lichtreflektor arbeiten, wie z.B. ansteuerbare Mikrospiegel. Als technische Lösung für ein Lichtquellen-Array kommen flächig angeordnete Strukturen auf Galliumnitrid (GaN) basierend in Frage. GaN ist als UV-Emitter z.B. aus der Fertigung kommerziell erhältlicher UV-LED's bekannt. Aus diesen Strukturen wird durch geeignete Verschaltung eine Matrix mitvielen unabhängig ansteuerbaren Elementen aufgebaut. Weiterhin ist ein entsprechend aufgebautes Mikrolaser-Array denkbar, wobei als laseraktives Medium z.B. GaN Verwendung finden kann.

Eine solche Vorrichtung kann beispielsweise aus einer Matrix emittierender Halbleiterelemente, die Licht einer Wellenlänge < 400 nm emittieren, wie es beispielsweise durch GaN-Leuchtdioden erfolgt. Wie erwähnt, kommt als Belichtungsmatrix auch ein entsprechend aufgebautes Mikrolaser-Array in Frage. Die Größe eines lichtemittierenden Elementes kann in einem Bereich zwischen 500 x 500 µm und 50 x 50 µm liegen. Jedes Matrixelement ist separat ansteuerbar. Bei einem Belichtungsvorgang, der Grundlage einer biochemischen Reaktion ist, emittiert zumindest eine Leuchtdiode Photonen innerhalb eines Wellenlängenbereiches unterhalb von 400 nm. Da die Vorrichtung vorzugsweise als Einheit zur Initiierung räumlich getrennter photochemischer Reaktionen in einem Reaktionsträger konzipiert ist, ist ein Füllgrad der Belichtungsmatrix mit lichtemittierenden Elementen von weniger als 75% notwendig.

Die Größe der Lichtquellenmatrix ist größer oder gleich der optischen Abbildung auf dem Reaktionsträger. Die ggf. erforderliche Verkleinerung der Abbildung wird bevorzugt durch Lichtwellenleitung in einem Glasfaserbündel (fused fiber optic taper), wahlweise auch durch geeignete Linsensysteme, realisiert. Der Einsatz von fused fibre optic tapers ist beispielsweise aus Nachtsichtgeräten bekannt.

Das Anordnungsmuster der UV-Leuchtdioden entspricht vorzugsweise dem Muster der Syntheseplätze im Reaktionsträger.

Der Aufbau der Belichtungskomponente (selbstleuchtende Lichtquellenmatrix) besteht somit aus einer Matrix, auf der UV-Leuchtdioden oder Mikrodiodenlaser in Zeilen und Spalten angeordnet sind. Durch Ansteuerung der einzelnen Lichtquellenelemente dieser Matrix wird ein spezifisches Belichtungsmuster erzeugt, welches dem Muster der Syntheseplätze im Reaktionsträger entspricht.

Die einzelnen Lichtquellenelemente werden beispielsweise zeilen- und spaltenweise angesteuert, wobei ein Pulsieren der einzelnen Leuchtdioden oder Laserelemente eintritt, d.h. es wird eine schwankende Lichtintensität emittiert. Ein ähnliches Verfahren der Ansteuerung ist beispielsweise bei LCD-Belichtungsmatrizen zu finden. Alternativ kann eine statische Ansteuerung der einzelnen Leuchtdioden der Matrix durch bistabile Kippschaltungen (Flip-Flops) oder DRAM's sowie sonstige geeignete Schaltungen erfolgen.

Unmittelbar an das Lichtquellen-Array kann sich eine Matrix aus optischen Mikroelementen (oder auch eine mechanische Lochmaske zur Streulichtunterdrückung) anschließen. Diese Komponente kann ihrerseits aus einer von mehreren miteinander verbundenen Schichten mikroskopischer, optischer Bauteile (z.B. Mikrolinsen) bestehen und wird zweckmäßigerweise direkt auf der Lichtquellenmatrix befestigt.

In einer Ausführungsform schließt sich unmittelbar an die mikrooptische Komponente ein "fused fiber optic taper" an, welches zur Verkleinerung des Beleuchtungsmusters im Verhältnis (Eintritt : Austritt) 1 : 1, 2 : 1, ..... 25 : 1 oder etwaigen Zwischenwerten dient. Hierbei können die einzelnen Fasern des Glasfaserbündels durch eine schwarze Ummantelung optisch voneinander abgschirmt sein.

Zwischen den einzelnen Baukomponenten der Vorrichtung kann sich ein fluidisches optisches Medium befinden. Die Einkopplung des erzeugten Belichtungsmusters in den Reaktionsträger kann ihrerseits über ein Glasfasebündel erfolgen, welches unmittelbar auf der Oberfläche des planaren Reaktionsträgers befestigt ist.

Der mögliche Aufbau des Reaktionsträgers und die Anordnung einer Lichtsensormatrix (Vielkanaldetektormatrix), die vorzugsweise in Form eines CCD-Chips vorgesehen ist, wird nachstehend noch erläutert.

Der Reaktionsträger wird auf der lichtemittierenden Seite der Lichtquellenmatrix angeordnet. Der Reaktionsträger ist zumindest auf der der Belichtungsmatrix zugewandten Seite optisch transparent. Dadurch kann in diesem Reaktionsträger, der z.B. ein optofluidischer Mikroprozessor sein kann, ein ortsaufgelöstes Belichtungsmuster erzeugt werden. Auf diese Weise kann in dem Reaktionsträger unter Verwendung einer geeigneten Photochemie innerhalb der einzelnen Reaktionsbereiche die Immobilisierung oder Synthese von Polymersonden ortsaufgelöst gesteuert werden.

Eine Vorrichtung zur Umsetzung des beschriebenen Verfahrens kann in einem sehr kompakten und platzsparenden Aufbau realisiert werden, in dem dann sowohl die Aktivierung der Synthese auf dem Reaktionsträger und damit die Dotierung der Reaktionsbereiche mit entsprechenden Polymersonden als auch die Detektion von Signalen nach Zugabe von Probenmaterial erfolgen kann.

Zwischen dem Reaktionsträger und der betreffenden Lichtsensormatrix kann sich ein spektrales Filter (Bandpaß- oder Langpaßfilter) befinden, welches bei der fluoreszenzspektroskopischen Detektion der an den BioChip (Reaktionsträger) gebundenen Analyten eine spektrale Separation des Signallichtes vom Anregungslicht ermöglicht. Die Verwendung eines Filterrades mit verschiedenen optischen Filtern erlaubt darüber hinaus die simultane Detektion von Analyten verschiedener Probenmaterialien, welche durch unterschiedliche Farbstoffe mit spektral weit auseinanderliegenden Fluoreszenzmaxima markiert worden sind.

Die Arbeitsweise der Lichtquellenmatrix (Belichtungsmatrix) und der betreffenden Lichtsensormatrix (z.B. CCD-Array) können entweder durch eine geeignete Hardware oder Software aufeinander abgestimmt werden. Wenn die einzelnen Elemente der Belichtungsmatrix auf einer Nanosekunden-Zeitskala geschaltet werden können, ohne z.B. "nachzuleuchten", so ist auch eine elektronische Synchronisation mit einer sogenannten "gegateten" CCD-Kamera über einen externen Frequenzgenerator möglich. Da die Fluoreszenzlebensdauer gebräuchlicher Farbstoffe gewöhnlich einige Nanosekunden beträgt, ist auf diese Weise bei der fluoreszenzspektroskopischen Detektion des Analyten eine zeitliche Separation des Anregungs- und Signallichtes möglich, so daß zeitaufgelöste Spektroskopie betrieben werden kann.

Eine weitere Klasse von erfindungsgemäß verwendbaren Belichtungsmatrizen stellen Matrixanordnungen von "Lichtventilen" oder steuerbaren Durchlicht-Modulatoren dar, welche ortsselektiv steuerbar sind, um Licht durchzulassen bzw. Licht nicht durchzulassen. Hierbei handelt es sich um elektronische Komponenten, bei denen das Licht einer Lichtquelle auf eine Matrix von steuerbaren Pixeln fällt. Jedes Pixel kann in Bezug auf seine Lichtdurchlässigkeit durch das elektronische Steuersignal moduliert werden. So entsteht eine steuerbare Lichtventilmatrix LVM. Um die Funktion des Lichtventils auszufüllen, müssen Teilederelektronischen Komponenten (u.a. die eigentlichen Elektroden) transparent sein. Zur Gruppe der Lichtventile zählt als bekanntester Vertreter das Liquid Cristall Display LCD. Lichtventile auf LCD-Basis sind weit verbreitet, als Mikroversion u.a. im Sucher von digitalen Videokameras und in Nachtsichtgeräten und als Makroversion zum Beispiel in Laptops oder als Bildschirm zu Personal Computern. Die Transmission im Dunkelzustand beträgt allerdings immer noch bis zu 10% der Lichtmenge, die von hinten eingestrahlt wird. LCDs sind für Wellenlängen transmittierten Lichtes oberhalb 400 nm verfügbar. Für Belichtung im UV-Bereich des Lichtes sind die enthaltenen Kristalle schlecht geeignet, u.a. aufgrund ihrer Eigenabsorption (siehe u.a. Microsystem Technologies 1997, 42-47, Springer Verlag). Für die Konfiguration von LVMs im UV-Bereich sind daher andere Substanzen als Füllung zwischen den transparenten Elektroden notwendig. Solche alternativen Substanzen sind z.B. aus sog. Suspended Particle Devices SPD bekannt (siehe u.a. US 5728251). Solche und andere Substanzen können mit der gleichen Elektrodenanordnung wie LCDs verwendet werden, es ist aber auch möglich, andere transparente Komponenten zu verwenden.

Bei dem erfindungsgemäßen Verfahren kann es vorgesehen sein, daß die Belichtung des Trägers durch pulsierende, kohärente, monochromatische, parallele oder/und gegebenenfalls in unterschiedlichen Ebenen fokussierbare Strahlung erfolgt.

Der Reaktionsträger bzw. BioChip kann beispielsweise eine Halbleiteroberfläche, eine Glasoberfläche oder eine Kunststoffoberfläche für die Beschichtung mit biologisch oder biochemisch funktionellen Materialien aufweisen, wobei es sich um eine Außenoberfläche oder/und um eine Innenoberfläche des Trägers handeln kann, letzteres, sofern der Träger zumindest teilweise ausgehöhlt, beispielsweise von Kanälen durchsetzt ist.

Vorzugsweise wird ein transparenter Träger verwendet, der optische Untersuchungen im Durchlichtverfahren ermöglicht.

Die vorbestimmten aktivierbare Bereiche können beispielsweise eine Fläche von 1 µm² bis 1 cm², insbesondere 100 µm² bis 1 mm² umfassen. Die vorbestimmten aktivierbaren Bereiche können von nichtaktivierten oder/und nichtaktivierbaren Bereichen umgeben sein.

Die Belichtungsmatrix kann ein für die vorbestimmten aktivierbaren Bereiche inhärentes Muster aufweisen, beispielsweise mit Stellen, die im Belichtungsmuster stets Abschattung bzw. Dunkelheit zur Folge haben.

Die biologischen oder biochemisch funktionellen Materialien werden vorzugsweise aus biologischen Substanzen oder mit biologischen Substanzen reaktiven Materialien ausgewählt, nämlich vorzugsweise aus Nukleinsäuren und Nukleinsäurebausteinen, insbesondere Nukleotiden und Oligonukleotiden, Nukleinsäureanaloga wie PNA und Bausteinen davon, Peptiden und Proteinen und Bausteinen davon, insbesondere Aminosäuren, Sacchariden, Zellen, subzellulären Präparationen, wie Zellorganellen oder Membranpräparationen, viralen Partikeln, Zellaggregaten, Allergenen, Pathogenen, pharmakologischen Wirkstoffen und diagnostischen Reagenzien.

Die biologisch oder biochemisch funktionellen Materialien werden vorzugsweise durch mehrstufigen Aufbau aus Monomer- oder/und Oligomerbausteinen auf dem Träger synthetisiert.

Die große Flexibilität des Verfahrens nach der Erfindung ermöglicht die Erzeugung einer umfangreichen Substanzbibliothek mit einer Vielzahl unterschiedlicher biologisch oder chemisch funktioneller Materialien auf dem Träger.

Die Aktivierung von vorbestimmten Bereichen umfaßt insbesondere eine Schutzgruppenabspaltung vom Träger selbst oder von darauf gebundenen Materialien oder Bausteinen davon.

Die Belichtungsmatrix ermöglicht eine flexible zeitliche Steuerung der Belichtungsabläufe, so kann die Belichtung mit einer Geschwindigkeit aus dem Bereich von beispielsweise 1 /10.000 bis 1000, insbesondere von 1/10 bis 100 Lichtmustern pro Sekunde erfolgen.

Gemäß einer bevorzugten Verfahrensvariante wird die Belichtung des Trägers mit einer Lichtsensormatrix, insbesondere einer CCD-Matrix überwacht und ggf. unter Berücksichtigung der dabei gewonnenen Informationen gesteuert. Vorzugsweise istdie Sensormatrix der Belichtungsmatrix zugewandt gegenüberliegend angeordnet, wobei der Träger zwischen Belichtungsmatrix und Sensormatrix positioniert ist, um Durchlicht-Beobachtung möglich zu machen. Alternativ können die Belichtungsmatrix, der Träger und die Sensormatrix auch zu einer Auflichtanordnung gruppiert werden.

Die Sensormatrix kann dazu herangezogen werden, eine automatische Erkennung und/oder gegebenenfalls Kalibrierung des jeweils verwendeten Trägers mittels einer der Sensormatrix nachgeschalteten Auswerteeinheit durchzuführen.

Bei einer Weiterbildung der Erfindung kann es vorgesehen sein, daß die auf dem Träger synthetisierten Materialien, insbesondere Polymere, wie Nukleinsäuren, Nukleinsäureanaloga und Proteine, abgelöst werden, um sie für bestimmte Zwecke zur Verfügung zu stellen. Unter diesem Aspekt kann das Verfahren quasi als Produktionsverfahren für biochemische Materialien genutzt werden. Dabei kann es vorgesehen sein, daß die Materialien in unterschiedlichen Bereichen in aufeinanderfolgenden Schritten abgelöst und als Bausteine zum weiteren Aufbau von Polymeren, insbesondere Nukleinsäure-Polymeren, eingesetzt werden.

Weitere Gesichtspunkte der Erfindung sind in den Ansprüchen 25 bis 40 angegeben, so insbesondere die Verwendung einer Belichtungsmatrix, die zur Erzeugung eines wahlweise einstellbaren Belichtungsmusters steuerbar ist, als Lichtquelle einer Lichtemissions-Detektionseinrichtung zur Detektion des optischen Verhaltens eines mit biologisch oder biochemisch funktionellen Materialien versehenden 2- oder 3-dimensionalen Testbereichs, wobei die Herstellung des Testbereichs vorzugsweise in der Lichtemissions-Detektionseinrichtung erfolgt.

Weiterhin sei noch auf einen Gesichtspunkt der Erfindung hingewiesen, gemäß dem eine steuerbare Belichtungsmatrix dazu verwendet wird, Reaktionsträger mit Zellen/Gewebeschnitten ortsaufgelöst zu belichten, um belichtungsabhängige Manipulationen vorzunehmen (lichtempfindliche Prozesse, wie Photosynthese, Manipulation von Retina-Präparaten, lichtabhängige neuronale Aktivität) oder Analysen durchzuführen (als 2D-FACS; cell-array, tissue-derived cell-array).

Gegenstand der Erfindung ist ferner eine Lichtemissions-Detektionseinrichtung nach einem der Ansprüche 41 - 43.

Dabei dient als Belichtungsmatrix bzw. Lichtquellenmatrix, eine hinsichtlich ihrer Lichtdurchlässigkeit ortsselektiv steuerbare Belichtungsmatrix, insbesondere eine Lichtventilmatrix, eine Reflexionsmatrix oder eine selbstleuchtende bzw. selbstemittierende Belichtungsmatrix.

Gemäß einer Ausführungsform der Lichtemissions-Detektionseinrichtung basiert die Belichtungsmatrix auf einer Lichtventil-Matrix (z.B. LCD-Matrix). In Kombination mit einer geeigneten Lichtquelle ermöglicht die Lichtventil-Matrix die Realisierung einer hochparallelen, hochauflösenden und ortsspezifischen Anregungslichtquelle und Inspektionslichtquelle, die aufgrund ihrer Flexibilität eine Vielzahl an Anwendungsmöglichkeiten eröffnet. Lichtventil-Matrizen sind durch ihren breiten Einsatz im elektronischen Konsumgüterbereich weit entwickelt und dadurch zuverlässig, billig und extrem klein. Wie bereits erläutert, ist eine mögliche Anwendung einer solchen Belichtungsmatrix der Ersatz der aufwendigeren Photolithographie (z.B. bei der photoaktivierten Oligosynthese bei der Herstellung von DNA-Chips) bei weniger hohen Auflösungen, wie beispielsweise bei einfachen Si-Chips oder den DNA-Chips.

Als Lichtsensormatrix kommt vorzugsweise ein CCD-Bildaufnehmer (CCD-Kamera-Chip) in Frage. Ordnet man diese beiden Chips einander gegenüberliegend an, so erhält man eine extrem kompakte, hoch parallele Anregungs-, Inspektions- und Detektionseinheit für eine noch größere Vielzahl von Anwendungen. Die zweidimensionale Lichtemissions-Detektionseinheit entwickelt ihr enormes Potential insbesondere durch das intelligente Zusammenspiel von flächiger Ansteuerung und flächiger Auslesung. Hier bietet die Leistungsfähigkeit moderner Rechner und Softwaresysteme enormes Anwendungs-und Entwicklungspotential, wobei man sowohl bei Hard- als auch bei der Software auf die vorhandenen Systeme zur Nutzung der Lichtventil-Matrix (z.B. LCD) als Mensch-Maschinen-Schnittstelle aufbauen kann. Bei Anwendungen als Kombination aus Lichtquelle und Detektor ist die lntensitätsempfindlichkeit (z.B. 264 Stufen bis 4096 bzw. bis mehrere 100 000 bei CMOS-CCDs) und die Unterscheidung von Farben (d.h. Wellenlängen) im CCD-Chip (z.B. Filtern von Peaks für rot, grün und blau oder andere Farben, je nach Filtern vor den Pixeln) für eine zweidimensionale Spektroskopie geeignet. Zwischen Belichtungsmatrix und Lichtsensormatrix wird an oder in dem Träger ein zu untersuchender/zu analysierender/anzuregender oder anderweitig gezieltmit Licht zu bestrahlender und synchron nach Lichterscheinungen zu untersuchender Gegenstand oder sonstiges Untersuchungsgut eingebracht. Es entsteht eine Art Sandwich-Aufbau aus Belichtungsmatrix, Träger bzw. Untersuchungsgegenstand und Lichtsensormatrix. Zwischen der Belichtungsmatrix und dem Untersuchungsgegenstand, ebenso wie zwischen dem Untersuchungsgegenstand und dem Lichtsensor-Matrixchip soll vorzugsweise nur ein minimaler Abstand bestehen, um die Abweichung (Streuung) des Lichtes vom betreffenden Pixel der Belichtungsmatrix zum gegenüberliegenden Pixel der Lichtsensormatrix zu minimieren.

Während der Syntheseschritte dient die Lichtemissions-Detektionseinrichtung auch als Detektor z.B. für Flüssigkeitsbewegungen und erlaubt eine integrierte Qualitätskontrolle bzw. Prozeßkontrolle. Dies wirkt sich positiv auf Qualtität und Ressourcenverbrauch aus und reduziert die Ausschußrate. Wenn keine Prozeßüberwachung bei der Synthese benötigt wird und die Detektion in einem getrennten System erfolgt, kann anstelle der Lichtsensormatrix z.B. auch eine Temperierungseinheit vorgesehen sein.

Durch die Anordnung einer hoch parallelen Belichtungsmatrix und einer hochparallelen Lichtsensormatrix entsteht eine breit einsetzbare, neuartige Inspektionseinheit, welche man auch als massive parallele Lichtschranke bezeichnen kann, die, wenn notwendig, auch noch die Vorteile einer quantitativen und qualitativen Anregung und Messung einschließt. Eine weitere Besonderheit ist die Möglichkeit, unterschiedlich farbiges (unterschiedliche Wellenlänge) Licht zu verwenden. Im Falle einer Lichtventilmatrix läßt sich beispielsweise die Anregungswellenlänge grundlegend durch die jeweilige Verwendung der geeigneten Hintergrundbeleuchtung der Lichtventilmatrix bestimmen.

Eine weitere Stärke der Lichtemissions-Detektionseinrichtung sind die fast unendlichen Möglichkeiten, welche sich aus der Kombination von gezielter Anregung und gezielter Detektion in Verbindung mit modernen Hochleistungsrechnern für die Ansteuerung und die Signalauswertung ergeben. Damit wird gerade für optische Nachweis- und Detektionsverfahren eine neue Technologieplattform geschaffen. Durch das "Durchtunen" der einzelnen Lichtpunkte im Zusammenspiel mit der CCD-Detektion und geeigneten Algorithmen zur Signalauswertung müßten kleinste Veränderungen in den einzelnen Meßpunkten in einer Lichtemissions-Detektionseinrichtung möglich sein. Im Bereich der DNA-Analytik wäre beispielsweise die direkte Detektion einer Hybridisierung in einem Reaktionsbereich denkbar.

Hinsichtlich der Bildverarbeitung und Steuerung der Systemkomponenten der Lichtemissions-Detektionseinrichtung kann man ggf. auf Hard- und Softwaretoolszurückgreifen. Beispiele sind Grafikkarten, Videoschnittkarten und dazugehörige Software.

Im Vergleich zu konventionellen photolithographischen Systemen bietet die Lichtemissions-Detektionseinrichtung die Möglichkeit einer extremen Miniaturisierung bei gleichzeitiger Funktionsintegration bei Verwendung als Synthese- und Analysesystem (ISA-System), insbesondere bei Verwendung einer Lichtventilmatrix, Reflektionsmatrix, eines Dioden-Arrays oder eines Laser-Arrays als Belichtungsmatrix und eines CCD-Bildwandlers als Lichtsensormatrix.

Besonders interessante Anwendungen einer Lichtemissions-Detektionseinrichtung nach der Erfindung werden im folgenden kurz dargelegt:
- Herstellen eines opto-fluidischen Reaktionsträgers, insbesondere nach einem Verfahren gemäß einem der Ansprüche 1 - 35. In diesem Zusammenhang eignetsich die Lichtemissions-Detektionseinrichtung nach der Erfindung insbesondere auch zur Herstellung eines Trägers für Analytbestimmungsverfahren, der eine Vielzahl von Kanälen, insbesondere Kapillarkanälen, umfaßt, wobei in den Kanälen eine Vielzahl von unterschiedlichen Rezeptoren immobilisiert ist bzw. zu immobilisieren ist. Ein solcher Träger ist beispielsweise in der DE 198 39 256.7 beschrieben (siehe Prioritätsbeleg DE 198 39 256.7 zur vorliegenden Anmeldung). Zur Herstellung eines solchen Trägers wird ein Trägerkörper mit einer Vielzahl von Kanälen bereitgestellt. Flüssigkeit mit darin enthaltenen Rezeptoren oder Rezeptorbausteinen wird durch die Kanäle des Trägerkörpers geleitet, wobei Rezeptoren oder Rezeptorbausteine an jeweils vorbestimmten Positionen in den Kanälen orts- oder/und zeitspezifisch immobilisiert werden. Das Immobilisieren kann in der Lichtemissions-Detektionseinrichtung nach der Erfindung durch Belichtung mittels der Belichtungsmatrix erfolgen. Der Aufbau eines Rezeptors am Trägerkörper kann durch mehrere aufeinanderfolgende Immobilisierungsschritte von Rezeptorbausteinen erfolgen.

Wie erwähnt, erfolgt die Photoaktivierung bei jedem Schritt direkt durch die Belichtungsmatrix. Im Falle der Verwendung eines LCD als Belichtungsmatrix kann die hierfür benötigte Wellenlänge von etwa 365 nm nicht erreicht werden, es sei denn der LCD ist als SPD ausgeführt.

Es ist denkbar, daß der Anwender sich seine hochparallelen Reaktionsträger selber erzeugt und direkt verwendet. Er lädt sich einfach die benötigten Daten (DNA-Sequenzen) von einer CD-ROM oder aus dem Internet und erzeugt in der Lichtemissions-Detektionseinrichtung (Aufbau analog einem externen Disketten-oder CD-ROM-Laufwerk) seinen individuellen DNA-Chip, benetzt ihn anschließend mit der Probe und liest die Signale aus.

Nutzt man z.B. jeden zweiten Pixel in dieser Anordnung für die Photoaktivierung, so kann man die Pixel dazwischen, welche innerhalb einer Kapillare (Mikrokanal in einem Reaktionsträger) des zumindest bereichsweise im wesentlichen transparenten Trägerkörpers liegen, für eine permanente Prozeßkontrolle verwenden. So kann man z.B. das Einströmen einer Luftblase zwischen zwei Fluiden in einer Kapillare individuell und dynamisch verfolgen. Auch ein Färben der Trägerfluide für G, A, C und T wäre denkbar, so daß die Anwesenheit der richtigen Oligonukleotide überprüfbar würde und eine Farbveränderung könnte eine Verschleppung signalisieren. Bei der anschließenden Detektion könnte wiederum eine ortsspezifische und wenn notwendig sogar farbspezifische Lichtanregung erfolgen. Hierdurch ergeben sich ganz neue Möglichkeiten für Nachweisverfahren, wie sie derzeit noch nicht vorhanden sind.

Mittels der Lichtemissions-Detektionseinrichtung können ferner Strömungsvorgänge in den Kapillaren in einem Glas- oder Kunststoffchip als Trägerkörper sowohl während der Produktion, sprich der Oügo-Synthese, als auch während der Analyse überwacht werden. Hierzu können z.B. Reinigungsluftblasen zwischen zwei Fluiden in den Kapillaren oder eine Färbung der einzelnen Fluide verwendet werden.

Für die lichtinduzierte Abspaltung von Schutzgruppen während der Synthese von DNA-Oligos auf dem Chip (Trägerkörper) kann die Belichtungsmatrix dienen, wobei z.B. mit einer Wellenlänge von 365 nm belichtet wird. Die benötigten Leistungen sind beispielsweise 14 mW pro cm². Eventuell sind auch Weiterentwicklungen der Synthesechemie möglich, die z.B. unterschiedliche Wellenlängen ausnutzen.

Die Detektion der Nachweisreaktion im Träger für Analytbestimmungsverfahren kann ebenfalls in der Lichtemissions-Detektionseinrichtung nach der Erfindung erfolgen. Wenn der Nachweis über Fluoreszenzmarker realisiert wird, müßte hierzu ggf. die Hintergrundbeleuchtung gewechselt werden (automatisch möglich). Gegebenenfalls kommen hier auch neue Detektionsverfahren zum Einsatz, welche erst durch die extrem flexible, individuelle Anstrahlung und Detektion des einzelnen Meßpunktes möglich werden.

In einer bevorzugten Ausführungsform umfaßt das Detektionsverfahren zur Bestimmung eines Analyten unter Verwendung eines mit biologischen oder biochemischen funktionellen Materialien beschichteten Reaktionsträgers, die folgenden Schritte:
(a) Bereitstellen eines Reaktionsträgers mit einer Vielzahl von unterschiedlichen ortsspezifisch gebundenen oder chemischen funktionellen Materialien,
(b) Zugeben einer gegebenenfalls vorbereiteten Probe, die den oder die zu bestimmenden Analyten enthält, Inkontaktbringen der Probe mit dem Reaktionsträger unter Bedingungen, bei denen eine Bindung des oder der zu bestimmenden Analyten an die trägergebundenen Materialien (Rezeptoren) erfolgt und gegebenenfalls anschließendes Waschen des Reaktionsträgers, und
(d) optisches Analysieren der Reaktionsbereiche im Rücklicht oder Durchlicht mittels Belichtungsmatrix und Sensormatrix.

Die Analytbestimmungsschritte (a) bis (c) können in den Syntheseprozeß integriert werden, so daß die Analyse unmittelbar nach Beendigung der Synthese durchgeführt wird. Dies ermöglicht die Verwendung der Analyseergebnisse eines vorherigen Synthesezyklus für die Auswahl der notwendigen trägergebundenen Materialien für die Reaktionsbereiche im nachfolgenden Reaktionsträger. Das Verfahren kann anschließend mit Schritt (a) fortgeführt werden, da das Analyseergebnis eine neue Auswahl der in den Reaktionsbereichen gebundenen Materialien bedingen kann.
- Eine weitere Anwendungsmöglichkeit für eine Lichtemissions-Detektionseinrichtung nach der Erfindung bezieht sich auf die Einbeziehung in ein Verfahren zur Bestimmung einer Vielzahl von Analyten in einer Probe, wie es in der deutschen Patentanmeldung 198 39 255.9 (siehe Prioritätsbeleg DE 198 39 255.9 zur vorliegenden Anmeldung) beschrieben ist. Dieses Verfahren zur Bestimmung einer Vielzahl von Analyten in einer Probe umfaßt die Schritte:
   (a) Inkontaktbringen der Probe mit
      (i) einer Vielzahl von Spezies von Mikropartikeln, wobei jede Spezies zum Nachweis von bestimmten Analyten geeignet ist, und eine bestimmte von anderen Spezies optisch unterscheidbare Codierung aufweist, und
      (ii) einer Vielzahl von löslichen, analytspezifischen Nachweisreagenzien, die eine signalgebende Gruppe tragen oder mit einer signalgebenden Gruppe bindefähig sind,
   (b) anschließendes Aufbringen der Mikropartikel auf einen Träger und
   (c) Bestimmen der Analyten durch optisches Erfassen der Codierung und der Menge des Vorhandenseins oder/und der Abwesenheit der signalgebenden Gruppen auf mindestens einer Spezies von individuellen Mikropartikeln auf dem Träger.
      Als Proben kommen insbesondere biologische Proben in Betracht. Bei den Mikropartikeln kann es sich um organische Partikel, wie organische Polymerlatices, oder um anorganische Partikel, wie Magnetpartikel, Glaspartikel etc., handeln.

Jede Spezies der vorzugsweise optisch transparenten Mikropartikel weist auf ihrer Oberfläche mindestens einen immobilisierten, unterschiedlichen, analytspezifischen Rezeptor auf. Die Codierung der Mikropartikel ist vorzugsweise eine Farbcodierung. Zur Bestimmung jedes Analyten wird mindestens ein lösliches, analytspezifisches Nachweisreagens verwendet.

Mittels der Lichtemissions-Detektionseinrichtung kann nach Aufbringen der Mikropartikel auf dem Träger und Einfügen des Trägers zwischen der Belichtungsmatrix und der Lichtsensormatrix eine statistische oder dynamische Anordnung der Mikropartikel auf dem Träger bestimmt werden, und zwar durch Bilderfassung mittels der Lichtsensormatrix.

Die auch als Beads oder Smart-Beads bezeichneten Mikropartikel repräsentieren in ihrer Gesamtheit eine Vielfalt an Meßpunkten. Die Lichtemissions-Detektionseinrichtung ermöglicht nicht nur die Lokalisation und Zuordnung der einzelnen Beads in ihrer Anordnung am Träger mittels der Lichtsensormatrix sondern darüber hinaus auch eine ebenso lokalisierte Beleuchtung. In dieser Kombination ist die Lichtemissions-Detektionseinrichtung daher besonders geeignet, um Bestandteile des auch als fraktalen Chip bezeichneten Trägers zu lokalisieren, zu identifizieren und mit entsprechender Software die notwendigen Daten zu liefern, um mit hoher Präzision den fraktalen Chip zu präparieren. Das Prinzip dieses Aufbaus und der umfassende Zugriff durch Beleuchtung und Detektion soll die Fehlerrate extrem niedrig halten.

Durch die Lichtemissions-Detektionseinrichtung können die Strömungsvorgänge der Smart-Beads in einem fraktalen Chip während der Analyse überwacht werden.

Die Auslesung der Informationen aus einem Smart-Bead-Array soll in der Lichtemissions-Detektionseinrichtung erfolgen, wobei die Anregungslichtquelle, also die Belichtungsmatrix, direkt über dem Smart-Bead-Array und die Lichtquellenmatrix direkt unter dem fraktalen Chip mit dem Smart-Bead-Array liegt. Durch diese möglichst kompakte Bauweise werden die Lichtlaufwege und damit auch die benötigte Lichtintensität ebenso wie Überlagerungseffekte benachbarter Smart-Beads minimiert. Auf die Verwendung einer aufwendigen, platzintensiven, lichtabsorbierenden und teueren Optik kann sowohl auf der Anregungs-, als auch auf der Detektionsseite verzichtet werden.

Eine weitere Variante ist eine vertikale Ausrichtung des Chips, so daß auch Gravitationskräfte für die Be- und Entladung des Chips mit den Smart-Beads genutzt werden können.

Als Sensormatrix kommt wiederum z.B. ein CCD-Chip in Frage. Ordnet man auf einer solchen CCD-Fläche von 25 x 37 mm mit 2000 x 3000 Farbpixeln Mikropartikel (Smart-Beads) mit einem Durchmesser von 60 µm zur Direktdetektion an, so erhält man mindestens 200 000 Mikropartikel (Smart-Beads). Jeder Mikropartikel überdeckt dabei ca. 120 quadratische Farbpixel mit 5 - 10 µm Kantenlänge. Damit erhält man 30-40 Farb- oder 120 Schwarzweißsignale pro Smart-Bead mit einer digitalen Lichtintensitätsabstufung von 256 bis 4096 (je nach CCD-Chip) diskreten Helligkeitsstufen je Schwarzweißpixel. Somit ist auf jeden Fall eine ausreichende Menge an Daten für eine statistische Signalverifizierung vorhanden.

Die Grenze der maximal synchron detektierbaren, unterschiedlich farbcodierten Smart-Beads liegt in der Möglichkeit der spezifischen Codierbarkeit(chemisches Limit der reproduzierbaren Farberzeugung) der Smart-Beads sowie in der Möglichkeit der optischen Erfassung der Farbunterschiede mit einem CCD-Chip. Teilt man die 256 Intensitätsstufen je Farbe (RGB Minimalanforderung) in 10 Stufen ein, so erhält man 25³ = 15 625 mögliche Farben, welche detektiert werden können. Baut man die Anzahl der Farbklassen durch weitere Farbfilter aus, so läßt sich die Zahl der detektierbaren Farben weiter erhöhen. Mit Vierfachfarbfiltern (z.B. RGB und Magenta) vor dem oben beschriebenen CCD-Chip ließen sich theoretisch 25 x 25³ = 390 625 Farben erkennen. Natürlich nur noch mit ca. 30 Vierfachfarbpixeln. Aufgrund der großen Fortschritte in der CCD-Technologie ist mit den aufgeführten Zahlen nur der absolute Standard dieser der Technik beschrieben. Neue Chips haben bereits 12 Bit (4096) Farbtiefe und erste Prototypen haben bereits auf der gleichen Fläche 81 Millionen Pixel. Damit ergibt sich auch für die beschriebene Applikation der CCD-Chip-Technologie ein großes Wachstumspotential und die parallele Detektion von 10⁶ individuellen Smart-Beads ist technisch machbar.

Gemäß einer Variante kann es vorgesehen sein, daß ein optisches Gitter zwischen Smart-Bead-Array (Fraktalchip) und CCD-Kamera-Chip vorgesehen ist.

Ferner kann es gemäß einer Variante vorgesehen sein, daß zwischen dem Smart-Bead-Array (Fraktalchip) und dem CCD-Kamera-Chip optische Elemente, insbesondere Abbildungselemente, vorhanden sind.
- Bei Anwendung der Lichtemissions-Detektionseinrichtung für High Throughput Screening (HTS)-Anlagen ließen sich beliebig viele der Lichtemissions-Detektions-Einheiten parallel aufbauen bzw. modular in ein Gerät integrieren. Die Bereitstellung der Oligos sowie der Waschflüssigkeit und der vorbereiteten Probe ist wiederum eine Frage der Ausführungsvariante. Hier gibt es zahlreiche Möglichkeiten von der Bereitstellung im Analysegerät bis zur Bereitstellung in der genau benötigten Menge direkt im Reaktionsträger, wo nur noch die Probe, z.B. nach einer PCR, zugegeben wird. Selbstverständlich ist auch die Integration der Probenvorbereitung in den Trägerchip denkbar. Bei einer Variante sollen die Fluide nur durch die Kapillarkräfte in betreffenden Kapillaren getrieben werden. Für die einzelnen Schritte muß lediglich das integrierte Ventil durch einen Stellmotor im Gerät verstellt werden (z.B. von außen durch einen Mikromotor oder einen Piezoantrieb, wenn die Lichtemissions-Detektionseinrichtung entsprechend miniaturisiert werden soll). Wenn die einzelnen Behälter oder Hohlräume im Chip nur mit einer Folie oder Membran oder einem entsprechenden Deckel verschlossen würden, könnte man bei mangelnder Kapillarkraft durch Druck von oben eine Pumpfunktion realisieren.

- Eine Verwendungsmöglichkeit einer Lichtemissions-Detektionseinrichtung nach der Erfindung könnte die Überwachung von langsamen Strömungsvorgängen bei der DünnschichtChromatographie (DSC) sein. Dabei kann ggf. auf die farbliche Markierung der Wanderung für die Detektion verzichtet werden und stattdessen eine direkte "In situ"-Kontrolle durch die kompakte und preiswerte Lichtemissions-Detektionseinrichtung durchgeführt werden.
- Eine Lichtemissions-Detektionseinrichtung nach der Erfindung kann auch in der Mikrosystemtechnik Verwendung finden als Inspektionseinheit etc.
- Bezüglich der Verwendung der Lichtemissions-Detektionseinrichtung zur Analytbestimmung in BioChips sei noch angemerkt, daß dort von dem Einsatz der CCD-Detektion eine linsenlose Signaldetektion erwartet werden sollte, die mindestens drei getrennte Wellenlängen-Peaks (Farben: rot, grün, blau) und 64 Intensitätsstufen unterscheiden kann.

Die Integration der differentiell lokalisierbaren Anregungslichtquelle in Form der Belichtungsmatrix wird mehrere Anregungswellenlängen (mindestens drei entsprechend der Farbgebung auf Monitoren) erlauben, so daß problemlos eine Verwendung unterschiedlicher Fluoreszenzmarker möglich ist.
- Eine weitere Anwendungsmöglichkeit für eine Lichtemissions-Detektionseinrichtung nach der Erfindung ist die Zytometrie und Untersuchung anderer ausreichend kleiner biologischer Objekte. Die Lichtemissions-Detektionseinrichtung überwacht allgemein eine 2D-Matrix durch lokalisierte Lichtschranken, wobei durch die Emissionsbreite und durch die hohe Empfindlichkeit und wellenlängenabhängige Detektion spektrometrische Prinzipien verwendet werden können.

Eine solche Matrix eignet sich damit hervorragend zur Untersuchung von Partikeln, die sich in einem flüssigen Medium zwischen "Detektor" und "Analyzer" befinden.

Als interessante Anwendung können als "Partikel" ganze Zellen untersucht werden. Im Gegensatz zu einer in einer 1D-Kapillare durchgeführten Zytometrie erfolgt dann eine parallele Klassifikation der Zellen entsprechend ihrer optisch erfaßbaren Parameter.

Denkbar sind die Bestimmung nach Größe, optische Eigenschaften, wie etwa Fluoreszenz (nach entsprechender Markierung mit spezifischen oder unspezifischen Färbungen, z.B. Antikörper respektive lipophiler Farbstoff) oder Bewegung z.B. bei Makrophagen, pathogenen oder anderen Einzellern o, ä. Auch die Untersuchung ausreichend kleiner Vielzeller ist möglich, z.B. eine ganze C. elegans-Population unter bestimmten experimentellen Bedingungen.
- Ein weiteres Anwendungsfeld für eine Lichtemissions-Detektionseinrichtung nach der Erfindung bietet die Gelelektrophorese. Dabei kann mittels einer Lichtemissions-Detektionseinrichtung die gelelektrophoretische Trennung von biologischem Untersuchungsmaterial, z.B. von DNA in Agarose-Gelen, online überwacht und ausgewertet werden, wenn Elektrophoresekammerund die Lichtemissions-Detektionseinrichtung entsprechend integriert werden. Der Benutzer könnte die Trennung z.B. auf dem Bildschirm verfolgen.

Dadurch wäre die Auswertung zum frühestmöglichen Zeitpunkt der Trennung ermöglicht, was evtl. eine enorme Zeitersparnis zur Folge hat. Die gesamte Gerätschaft könnte aufgrund der sensitiven und hochauflösenden Lichtemissions-Detektionseinrichtung wahrscheinlich deutlich kleiner konzipiert werden, als dies zur Zeit der Fall ist, da die meisten Gelelektrophoresen primär mit dem bloßen Auge beurteilt werden, und erst sekundär ein kleiner Teil der Trennungen unter einem Scanner ausgewertet wird. Aus dieser Verkleinerung folgt eine verbesserte Kühlung, die wiederum eine höhere Spannung und damit raschere Trennung ermöglicht. So ist eine weitere Beschleunigung des Vorgangs denkbar und insgesamt eine große Zeitersparnis (in Analogie zur Kapillarelektrophorese erscheint eine Beschleunigung um den Faktor 10 durchaus realistisch, bei reduziertem Material- und Analytverbrauch) möglich.

Eine mögliche Erweiterung ist die automatische Entnahme von Material aus dem online vermessenen Elektrophoresegel, z.B. durch einenangeschlossenen Miniroboboter/computer gesteuerten Apparat.

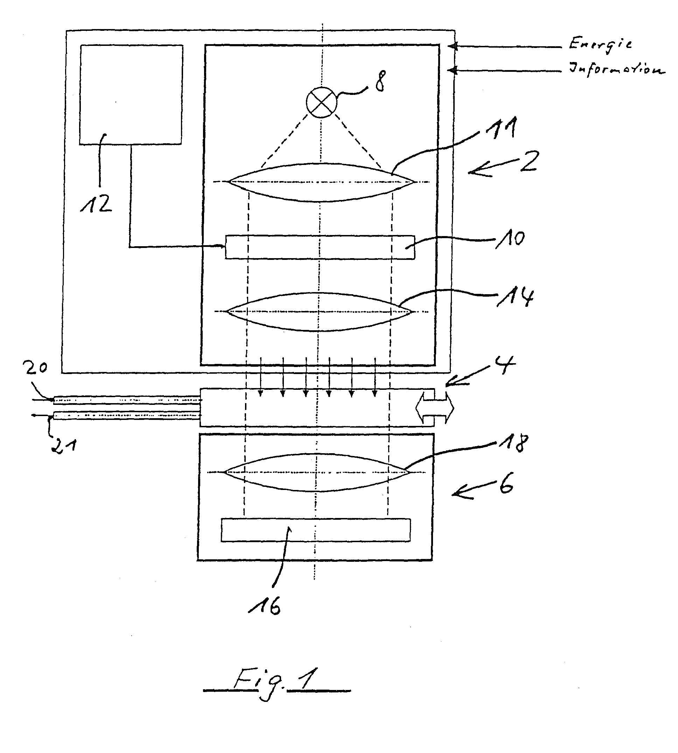

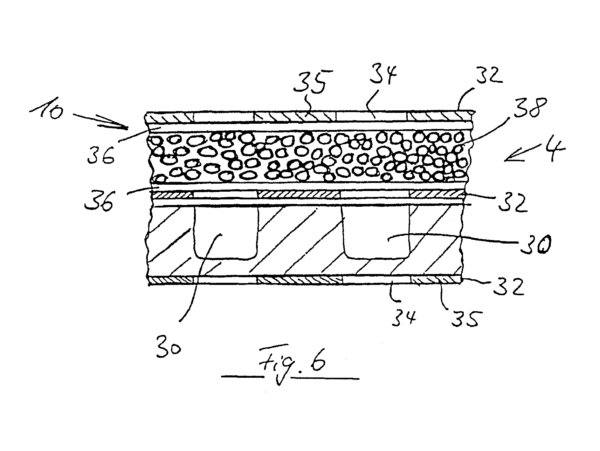

Einige Aspekte der Erfindung werden im folgenden unter Bezugnahme auf die Figuren erläutert. Die Figuren 1 - 5 zeigen in schematischer Darstellung unterschiedliche Ausführungsbeispiele für Vorrichtungen zur Herstellung/Manipulation/Untersuchung eines mit biologisch oder chemisch funktionellen Materialien beschichteten Trägers (BioChip). Fig. 6 zeigt eine Schnittdarstellung eines Teils eines Trägers mit integrierter Belichtungsmatrix.

Fig. 7 zeigt in einer stark schematisierten Darstellung ein Ausführungsbeispiel einer Lichtemissions-Detektionseinrichtung nach der Erfindung.

Die Fig. 8 bis 11 zeigen in einer schematischen Darstellung Vorrichtungen nach der Erfindung mit selbstleuchtenden Belichtungsmatrizen.

Fig. 1 zeigt eine erste Ausführungsform einer Anordnung zur Herstellung eines BioChips oder/und zur Manipulation oder/und zur Untersuchung darauf immobilisierter biologisch oder biochemisch funktioneller Materialien.

Die Anordnung nach Fig. 1 kann begrifflich in drei Funktionsbaugruppen oder Systemmodule 2, 4, 6 unterteilt werden. Der nachstehend auch als programmierbare Lichtquellenmatrix bezeichnete Systemmodul 2 umfaßt wenigstens eine Lichtquelle 8, wenigstens eine Belichtungsmatrix 10, die zur Erzeugung eines wahlweise einstellbaren Belichtungsmusters steuerbar ist, und einen Steuercomputer 12, bei dem es sich beispielsweise um einen programmierbaren Singlechip-Mikroprozessor handeln kann, welcher über eine betreffende Schnittstelle mit einem externen Rechner bedarfsweise kommunizieren kann, und dazu dient, die Belichtungsmatrix 10 nach einem betreffenden Programm zu steuern. Alternativ kann die Steuerung der Belichtungsmatrix von einem externen Rechner, z.B. Personal Computer, aus erfolgen. Das Systemmodul 2 kann ferner optische Elemente 11, 14 umfassen, bei denen es sich um Linsen, Blenden, Masken oder dergleichen, handeln kann und die gegebenenfalls auswechselbar angeordnet sind.

Der zweite Systemmodul 4 ist der auswechselbare Träger oder BioChip, der von der programmierbaren Lichtquellenmatrix 2 belichtet werden soll. Bei dem dritten Systemmodul 6 handelt es sich um eine Lichtdetektionseinheit, die vorzugsweise eine Matrix aus Lichtsensoren 16 umfaßt. Vorzugsweise handelt es sich bei der Matrix 16 um einen insbesondere farbtüchtigen CCD-Sensorchip, der für Spektral- und intensitätsaufgelöste, ortsselektive Messungen heranziehbar ist. Gegebenenfalls kann auch der Systemmodul 6 optische Elemente 18, wie Linsen, Blenden, Masken oder dergleichen enthalten.

Die Lichtsensormatrix 16 ist der Belichtungsmatrix 10 zugewandt gegenüberliegend angeordnet, wobei sich der Träger 4 im (Durchlicht-) Strahlengang zwischen der Belichtungsmatrix 10 und der Lichtsensormatrix 16 befindet.

In Beispielsfall nach Fig. 1 handelt es sich bei der Belichtungsmatrix 10 um eine elektronisch steuerbare optische Komponente, deren Transparenz ortsaufgelöst nach Maßgabe der Auflösung der Matrix, also der Anordnung und Größe der die Matrix bildenden und gezielt adressierbaren Matrixelemente, steuerbar ist, und zwar vorzugsweise zwischen zwei Zuständen, nämlich dem im wesentlichen opaken Zustand und einem Zustand maximaler Durchlässigkeit für das Licht der Lichtquelle 8. Man kann die Belichtungsmatrix 10 daher als elektronisch ansteuerbare Maske in Durchlicht-Anordnung betrachten. Je nach Ansteuerung durch den Steuerrechner 12 erzeugt die Belichtungsmatrix 10 ein Belichtungsmuster, mit dem der Träger 4 ortsselektiv belichtet wird. Bevorzugt wird als Belichtungsmatrix 10 in der Anordnung nach Fig. 1 eine Lichtventil-Matrix (LCD-Matrix mit SPD-Füllung) verwendet. Grundsätzlich können auch andere ortsaufgelöst steuerbare Lichtventilanordnungen, z.B. Mikroplatten, Mikroschieber, usw. zur Verwirklichung einer Belichtungsmatrix 10 der in Fig. 1 gezeigten Art herangezogen werden.

Der Detektionsmodul 6 kann zu seiner Steuerung und zur Verarbeitung der von ihm bereitgestellten Meßinformationen mit dem Computer 12 oder gegebenenfalls mit einem externen Computer, z.B. Personal Computer, in Verbindung stehen.

Die Systemmodule 2 und 6 sind vorzugsweise an einem in Fig. 1 nicht gezeigten gemeinsamen Halter angeordnet und gegebenenfalls relativ zueinander justierbar. Der Halter weist ferner eine Schiebeführung oder dergleichen auf, mittels der die auswechselbaren Träger 4 jeweils in die Position gemäß Fig. 1 auf einfache Weise eingebracht und aus dieser Position zur Herausnahme des betreffenden Trägers 4 auch wieder entfernt werden können.

Die Anordnung nach Fig. 1 kann in bevorzugter Weise dazu herangezogen werden, einen betreffenden Träger 4 ortsselektiv mit biologisch oder biochemisch funktionellen Materialien zu beschichten. Hierzu wird ein Träger 4 herangezogen, der eine Oberfläche mit photoaktivierbaren Gruppen aufweist. Beispiele geeigneter Träger sind u.a. in der deutschen Patentanmeldung 198 39 256.7angegeben. Die programmierbare Lichtquellenmatrix 2 wird dazu verwendet, ein Belichtungsmuster auf der mit photoaktivierbaren Gruppen versehenen Trägeroberfläche zu erzeugen, um die photoaktivierbaren Gruppen in vorbestimmten Bereichen zu aktivieren, die nach Maßgabe des Belichtungsmusters dem Licht der Lichtquelle 8 ausgesetzt sind. Der Oberfläche (im Beispiel einer Innenoberfläche des Trägers) können über den Zulauf 20 betreffende Reagenzien zugeführt werden, welche gewünschte biologisch oder biochemisch funktionelle Materialien oder Bausteine für solche Materialien enthalten, die dann an den vorbestimmten Bereichen binden können. Mit 21 ist eine Ablaufleitung für die Reagenzien bezeichnet.

Die biologisch oder biochemisch funktionellen Materialien oder Bausteine können ihrerseits mit photoaktivierbaren Gruppen versehen sein, die in einem etwaigen folgenden Aktivierungsschritt bereichsweise nach Maßgabe des dann gewählten Belichtungsmusters aktiviert werden können, um in einem weiteren Bindeschritt biologisch oder biochemisch funktionelle Materialien oder Bausteine für solche Materialien entsprechend den eingesetzten Reagenzien zu binden. Nicht aufgeführt wurden vorstehend etwaige Waschschritte zur Ausspülung der zuletzt verwendeten Reagenzien vor dem jeweiligen nächsten Belichtungsschritt. Je nach Aktivierungswellenlänge der photoaktivierbaren Gruppen kann es sich bei der auswechselbaren Lichtquelle 8 um eine jeweilige Strahlungsquelle handeln, die im Infrarotbereich, im sichtbaren Bereich, im ultravioletten Bereich oder/und im Röntgenbereich emittiert.

Belichtungs-, Wasch- und Bindungsschritte können in gezielt gesteuerter Weise wiederholt werden, um beispielsweise ein hochdichtes Mikro-Array aus Biomolekülen, wie z.B. DNA, RNA oder PNA zu erzeugen.

Für derartige Anwendungen ist der Lichtdetektionsmodul 6 nicht unbedingt erforderlich; er läßt sich jedoch in zweckmäßiger Weise zur Online-Qualitätskontrolle der Prozesse nutzen, die lichtabhängig in oder auf dem Träger 4 ablaufen, also z.B. für die Überwachung einer in situ-Synthese von Biomolekülen für die Herstellung eines Mikro-Arrays. Die Lichtsensormatrix 16 ermöglicht eine ortsaufgelöste Überwachung der lichtabhängigen Prozesse über optische Signale.

Der Lichtdetektionsmodul 6 kann allgemein zur Eichung oder Kalibrierung des Systems vor einer Synthese oder Analyse oder sonstigen Reaktionen bzw. Manipulationen auf dem oder im Träger herangezogen werden.

Die Lichtsensormatrix 16 kann ggf. auch für eine Typenerkennung verwendet werden, bei der z.B. ein für bestimmte Anwendungen bestimmter Träger oder Chip-Körper automatisch erkannt wird und die Reaktionen und Einstellungen während folgender Prozesse automatisch angepaßt werden.

Durch Verwendung optischer Elemente 14 kann das zweidimensionale Belichtungsmuster ggf. in einer oder mehreren bestimmten Ebenen in oder auf dem Reaktionsträger fokussiert werden. Auch eine Verschiebung der Fokussierebene während eines Prozesses ist denkbar.

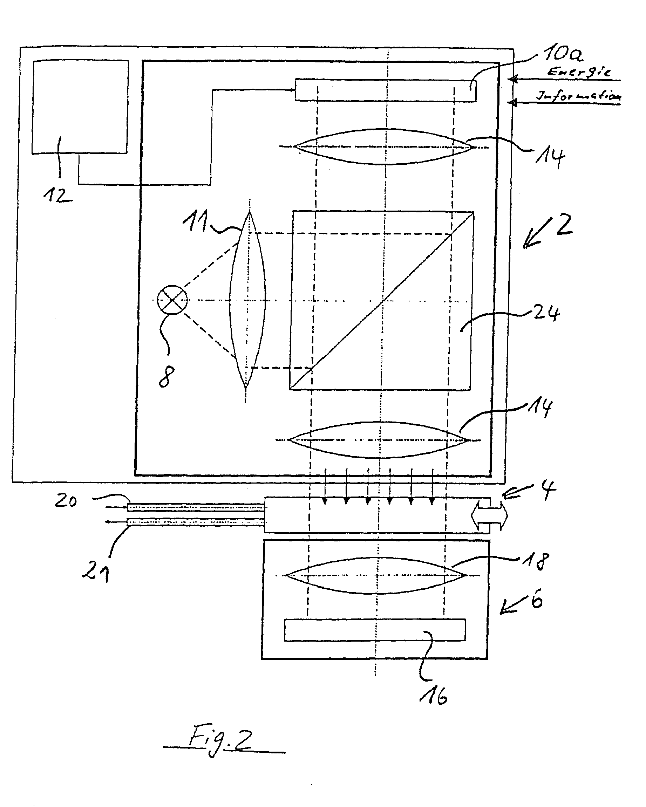

Fig. 2 zeigt in einer schematischen Darstellung eine zweite Ausführungsform einer Anordnung zur Herstellung, Untersuchung und/oder Manipulation eines Reaktionsträgers. Elemente in Fig. 2 - 6, die von ihrer Funktion her Elementen in Fig. 1 entsprechen, sind mit jeweils korespondierenden Bezugszeichen gekennzeichnet, so daß diesbezüglich auf die Beschreibung des ersten Ausführungsbeispiels verwiesen werden kann. Bei der Ausführungsform nach Fig. 2 ist eine elektronisch ansteuerbare Reflexionsmatrix 10a als Belichtungsmatrix vorgesehen. Als elektronisch ansteuerbare Reflexionsmatrix 1 0a kann beispielsweise ein hochauflösender Flächenlichtmodulator mitviskoelastischer Steuerschicht und Spiegelschicht verwendet werden. Derartige Flächenlichtmodulatoren mitviskoelastischen Steuerschichten sind beispielsweise in dem Datenblatt mit dem Titel "Lichtmodulatoren mitviskoelastischen Steuerschichten" erläutert, welches vom Fraunhofer-lnstitut für mikroelektronische Schaltungen und Systeme, D 01109 Dresden, herausgegeben wurde (Angaben daraus auf Seiten 44-47 der vorliegenden Anmeldung). Ein derartiger Flächenlichtmodulator erlaubt die Erzeugung eines ortsaufgelösten Belichtungsmusters zur Belichtung des Reaktionsträgers.

Als elektronisch ansteuerbare Reflexionsmatrix 10a kann alternativ auch ein Flächenlichtmodulator mit einem oder mehreren mikromechanischen Spiegelarrays verwendet werden, wie er in dem Datenblatt mit dem Titel "Lichtmodulatoren mit mikromechanischen Spiegelarrays" erläutert ist, welches vom Fraunhofer Institut für mikroelektronische Schaltungen und Systeme, D 01109 Dresden, herausgegeben wurde (Angaben daraus auf Seiten 48-52 der vorliegenden Anmeldung). Reflexions-Flächenlichtmodulatoren sind ferner von der Fa. Texas Instruments entwickelt worden.

Ganz allgemein eignen sich solche elektronisch steuerbare Spiegelmatrizen in CMOS-40V-Technik sehr gut für die Belange der vorliegenden Erfindung, da sie in einem weiten Spektralbereich, insbesondere auch in UV-Sepktralbereich des Lichtes eingesetzt werden können, um die gewünschten Belichtungsmuster zu erzeugen. Dies gilt nicht für UV-empfindliche Spiegelmatrizen in z.B. 5V-Technik.

Bei der Strahlengangführung gemäß Fig. 2 ist noch ein Lichtumlenkelement 24 erforderlich, bei dem es sich beispielsweise um einen teildurchlässigen Spiegel handelt, der das von der Lichtquelle 8 her kommende Licht zur Reflexionsmatrix 10a hin umlenkt und das von der Reflexionsmatrix 10a zurückreflektierte Licht nach unten hin zu dem Reaktionsträger 4 durchläßt, so daß auf dem Reaktionsträger 4 oder gegebenenfalls in dem Reaktionsträger 4 das nach Maßgabe der Ansteuerung der Reflexionsmatrix 10a erzeugte Belichtungsmuster zur Photoaktivierung, Analyse oder Manipulation von biochemischen Vorgängen genutzt werden kann.

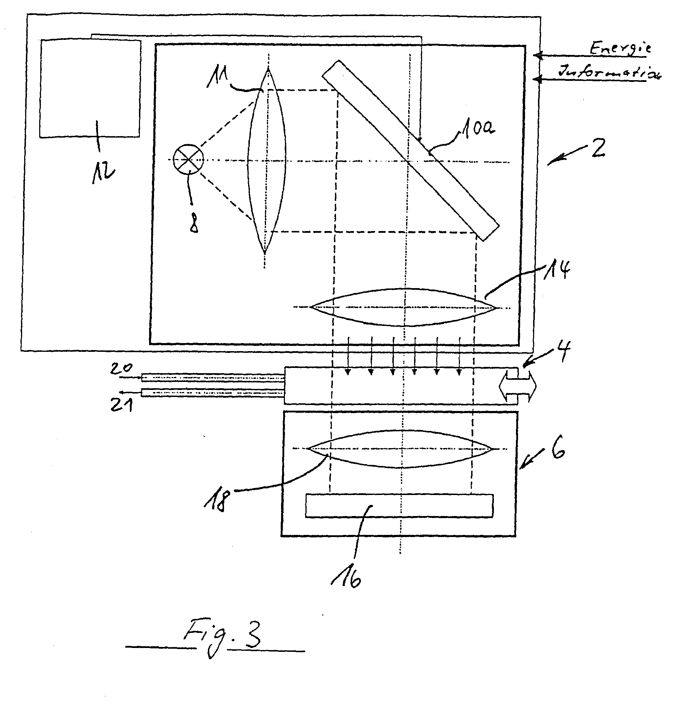

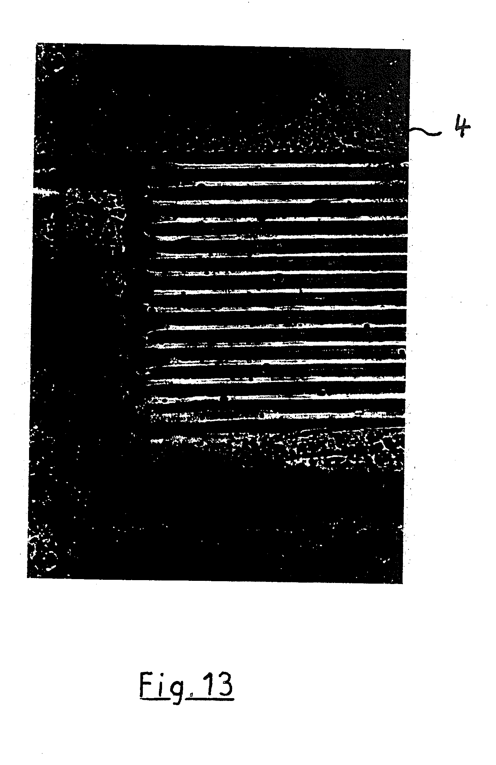

Fig. 3 zeigt eine Variante der Ausführungsform nach Fig. 2, wobei die Ausführungsform der Fig. 3 einen Strahlengang aufweist, bei dem auf das in Fig. 2 mit 24 bezeichnete Umlenkelement verzichtet werden kann, da die steuerbare Reflexionsmatrix 10a so angeordnet ist, daß sie von der Lichtquelle 8 kommendes Licht nach Maßgabe des gewählten Belichtungsmusters zum Reaktionsträger 4 hin umlenken kann. Bei Verwendung eines Aufbaus entsprechend der Variante von Fig. 3 sieht man in Fig. 13 die Abbildung eines Trägers mit mäanderförmigem Kanal. Dabei befinden sich zwischen Träger und CCD-Sensor keinerlei optische Elemente, es handelt sich um linsenlose Direktdetektion. Als Lichtquelle dient in diesem Fall ein Laser.

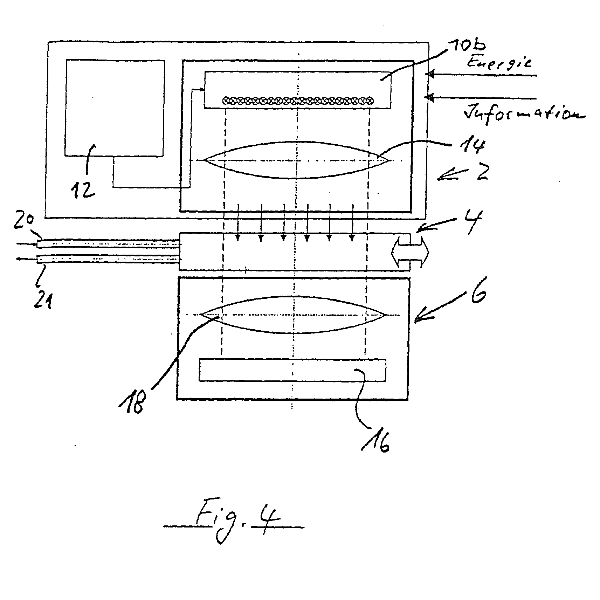

Fig. 4 zeigt in einer schematischen Darstellung eine weitere Ausführungsform einer Anordnung zur Herstellung, Untersuchung oder/und Manipulation eines Trägers nach der vorliegenden Erfindung. Bei der Ausführungsform nach Fig. 4 wird als Belichtungsmatrix eine Matrixanordnung 10b aus Lichtquellen, beispielsweise ein Mikrolaser-Array oder ein Mikrodioden-Array, verwendet. Es finden zur Zeit Entwicklungen statt, die darauf zielen, eine Vielzahl von mikroskopisch kleinen Halbleiterlasern als winzige, leistungsfähige Lichtquellen auf einem einzigen Chip unterzubringen. Ein derartiger steuerbarer "Licht-Chip" könnte als Matrix 10b herangezogen werden. Bezüglich Literatur zum Hintergrund der "Lichtchips" kann z.B. auf die Zeitschriften: Nature 3, 97, S. 294 - 295, 1999 und MPC-Spiegel 4/98, S. 13 - 17 verwiesen werden.

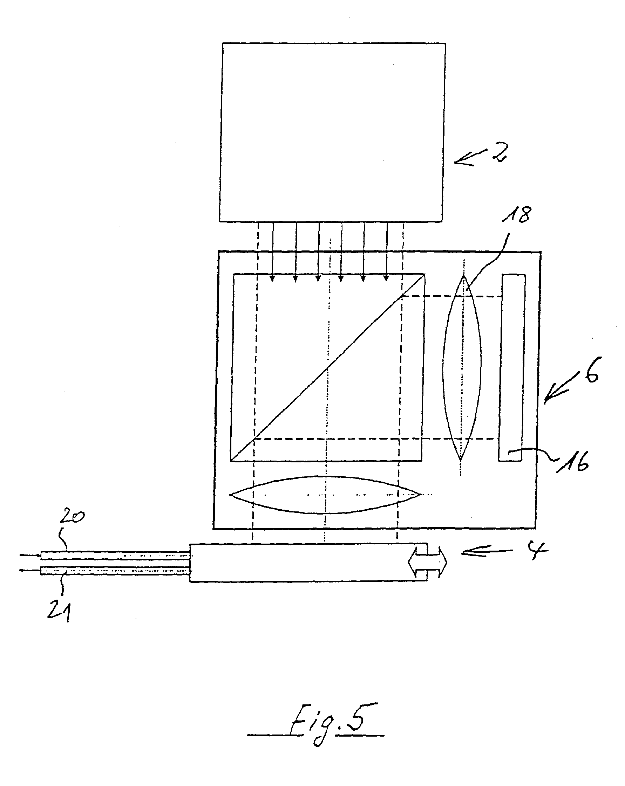

Fig. 5 zeigt eine Anordnung, bei der der Detektionsmodul 6 mit Sensormatrix 16 für Auflicht- bzw. Rücklichtbeobachtung des Reaktionsträgers 4 eingerichtet ist.

Sämtliche Anordnungen nach den Figuren 1-5 können als Lichtemissions-Detektionseinrichtung zur Detektion des optischen Verhaltens eines mit biologisch oder biochemisch funktionellen Materialien versehenen Testbereichs eines Trägers verwendet werden. Dies kann in einer Weise geschehen, wie es in der deutschen Patentanmeldung 198 39 254.0 offenbart ist.

Fig. 6 zeigt einen Schnitt durch eine Ausführungsform eines Trägers 4 nach der Erfindung, wobei sich diese Ausführungsform dadurch auszeichnet, daß die Belichtungsmatrix 10 Bestandteil des Trägerkörpers 4 ist. Als Belichtungsmatrix wird in diesem Fall vorzugsweise eine Lichtventil-Matrix verwendet, die zusammen mit ihrem Chipträger 4 entsorgt werden kann, nachdem der Träger nicht mehr gebraucht wird.

Im Beispielsfall der Fig. 6 weist der Trägerkörper 4 Kapillarkanäle 30 auf, deren Wände als Präparationsoberfläche für die Beschichtung mit biologisch oder biochemisch funktionellen Materialien dienen. Die Kanäle 30 können selektiv mit den betreffenden Reagenzien beschickt werden. In Fig. 6 sind folgende Einzelheiten zu erkennen: Begrenzungsschichten 32 mit lichtdurchlässigen und lichtundurchlässigen Bereichen 34 bzw. 35, transparente Elektroden 36 mit dazwischen eingeschlossenen und von den Elektroden 36 zu beeinflussenden SPD-Teilchen (suspended particles) oder alternativ Flüssigkristallen 38.

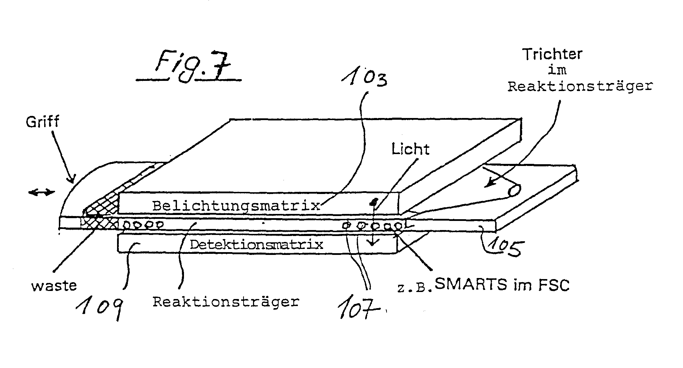

Fig. 7 zeigt in einer stark vereinfachten schematischen Darstellung eine Lichtemissions-Detektionseinrichtung nach der Erfindung in Form einer Sandwich-Struktur aus Lichtventilmatrix 103 (zweidimensionales Flüssigkristall-Belichtungselement), transparentem Probenträger 105 mit darin befindlichem Probenmaterial 107 und CCD-Matrix 109 (Bildaufnehmer). Lichtventilmatrix 103 und CCD-Matrix 109 sind von einer gemeinsamen (nicht gezeigten) Steuereinrichtung aus ansteuerbar, beispielsweise um einander zugeordnete Matrixelemente der Lichtventilmatrix und der CCD-Matrix gleichzeitig aktiv zu schalten.

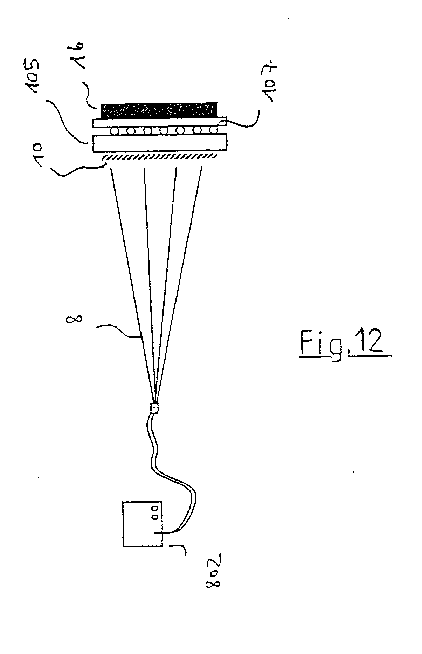

Die gezeigte Anordnung eignet sich z.B. zur Messung der optischen Absorption des Probenmaterials 107 in dem transparenten Träger 105. Bei dem Probenmaterial 107 kann es sich beispielsweise um Mikropartikel (Smart-Beads) handeln. Eine solche anordnung ist in Fig. 12 gezeigt. Hier ist als transparenter Träger 105 eine kommerziell erhältliche Neubauer-Zellzählkammer mit farbigen Mikropartikeln 107 gefüllt worden. Bei Belichtung mit einer Lichtquelle 8, bestehend aus Kaltlichtquelle mit Faserauskopplung und Lochblende (802), können farbige Mikropartikel mit dem CCD-Sensor detektiert und die Farbe durch die Absorption bestimmt werden (in schwarzweiß nicht zu sehen). Nach Markierung der Mikropartikel mit Fluoreszenzfarbstoffen lassen sich diese mit geeigneter Lichtquelle in gleicher Weise durch den CCD-Sensor detektieren.

Nicht zu erkennen in Fig. 7 sind Mittel zur Positionierung der Lichtventilmatrix relativ zur CCD-Matrix. Hierbei könnte es sich beispielsweise um Mittel zum Verschwenken der Lichtventilmatrix 103 relativ zur CCD-Matrix 109 handeln. Selbstverständlich können zahlreiche andere Möglichkeiten gewählt werden, um die Lichtventilmatrix 103 und die CCD-Matrix 109 lagerichtig zueinander zu positionieren und ggf. aneinander zu fixieren.

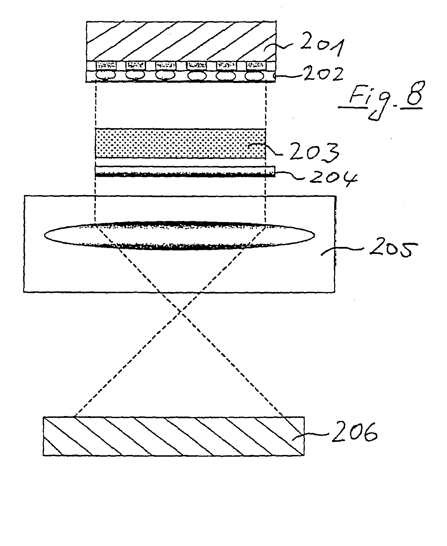

Fig. 8 zeigt eine weitere Anordnung zur ortsaufgelösten photochemischen Synthese von Polymersonden auf einem Träger und/oder zur Manipulation und/oder zur Untersuchung immobilisierter, biologischer, biochemisch funktioneller Materialien. Die Anordnung nach Fig. 8 kann begrifflich in die drei Funktionsbaugruppen 201, 203 und 206 unterteilt werden. Hierbei bildet das Systemmodul 201 eine Belichtungsmatrix z.B. in Form einer LED-Matrix, die softwaregesteuert ein Beleuchtungsmuster erzeugt, welches in dem Träger 203 die ortsaufgelöste photochemische Synthese von Polymersonden induzieren kann. Die Ankopplung des Beleuchtungsmusters in dem Träger 203 erfolgt mit Hilfe der Systemkomponente 202. Hierbei kann es sich beispielsweise um eine mechanische Maske mit einer Lochblende für jedes LED-Matrixelement handeln. Besser geeignet ist die Verwendung mikrooptischer Bauelemente, wie z.B. Mikrolinsen. Desweiteren kann es sich hierbei auch um ein "fused fiber optic taper" handeln, durch welches die Divergenz des von den einzelnen Lichtquellenelementen emittierten Lichtes erheblich vermindert werden kann. Gegenüber dem Träger 203 befindet sich auf der Austrittsseite des Lichtes ein optischer Vielkanaldetektor (vorzugsweise ein CCD-Sensor bzw. eine CCD-Kamera). Die Arbeitsweise dieser CCD-Kamera wird mit Hilfe einer geeigneten Hardware oder Software mit dem Betrieb der Belichtungsmatrix 201 abgestimmt. Zur Steuerung wird vorzugsweise ein Personal Computer verwendet, der die Belichtungsmatrix und die CCD-Kamera ansteuert. Zur Steuerung kann alternativ oder zusätzlich auch ein Frequenzgenerator herangezogen werden, mit dem die Belichtungsmatrix 201 und der dann "gegatete" CCD-Chip 206 elektronisch miteinander synchronisiert werden. Die letztere Ausführungsform wird inbesondere eine fluoreszenzspektroskopische Detektion der an den Träger 203 gebundenen Analyten ermöglichen, ohne daß zusätzliche frequenzselektive Elemente zwischen dem Träger 203 und der Detektormatrix 206 eingebracht werden müssen. Wie oben bereits erwähnt wurde, ist eine Voraussetzung für diese Methode jedoch, daß die einzelnen Elemente der Belichtungsmatrix im Zeitbereich weniger Nanosekunden ein- bzw. ausgeschaltet werden können. Alternativ hierzu kann ein optisches Filter 204 bei der fluoreszenzspektroskopischen Detektion eine spektrale Separation des Anregungs- und Signallichtes ermöglichen. Bei Verwendung eines Filterrades 204 ist es darüber hinaus möglich, Analyten verschiedener Probenmaterialien, die mit Fluoreszenzfarbstoffen deutlich unterschiedlicher Emissionsmaxima markiert worden sind, simultan auf dem gleichen Träger 203 zu analysieren. Die ortsselektive Abbildung des vom Träger 203 emittierten Signallichtes auf der Detektormatrix 206 kann optional mit der Baukomponente 205 erfolgen. Hierbei kann es sich entweder um ein abbildendes Linsensystem oder um ein "fused fiber optic taper" handeln.

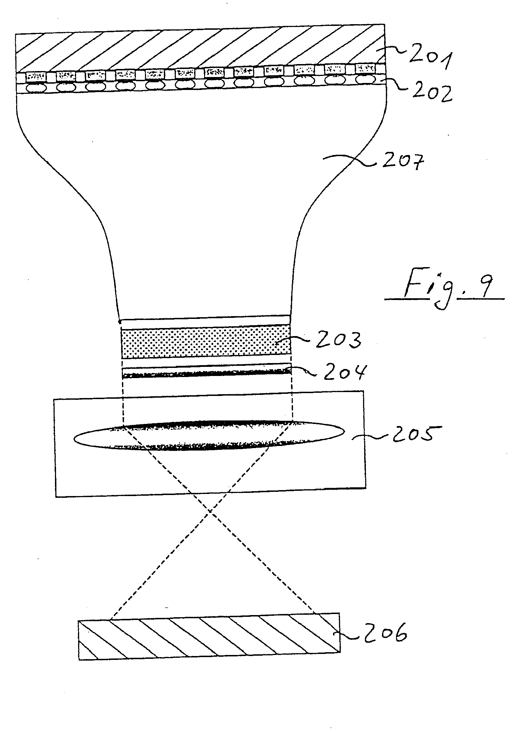

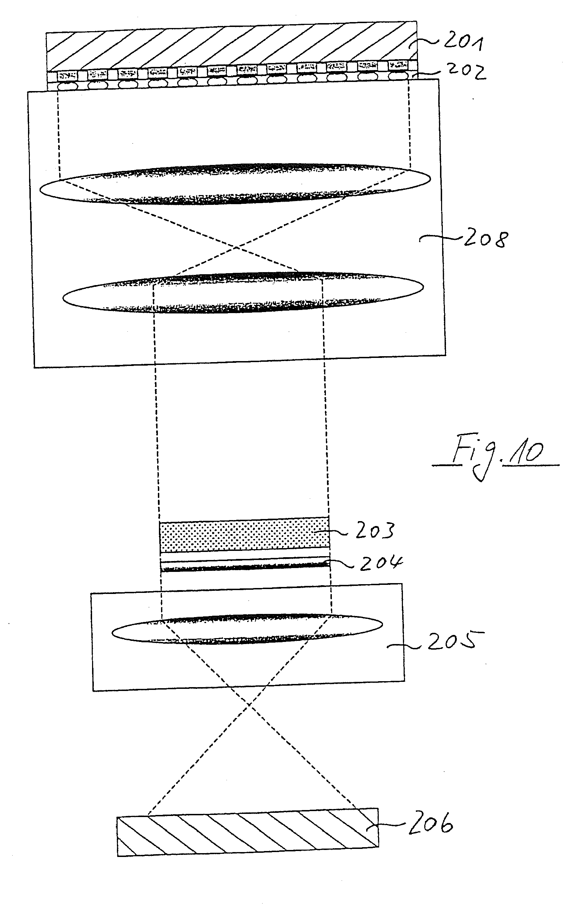

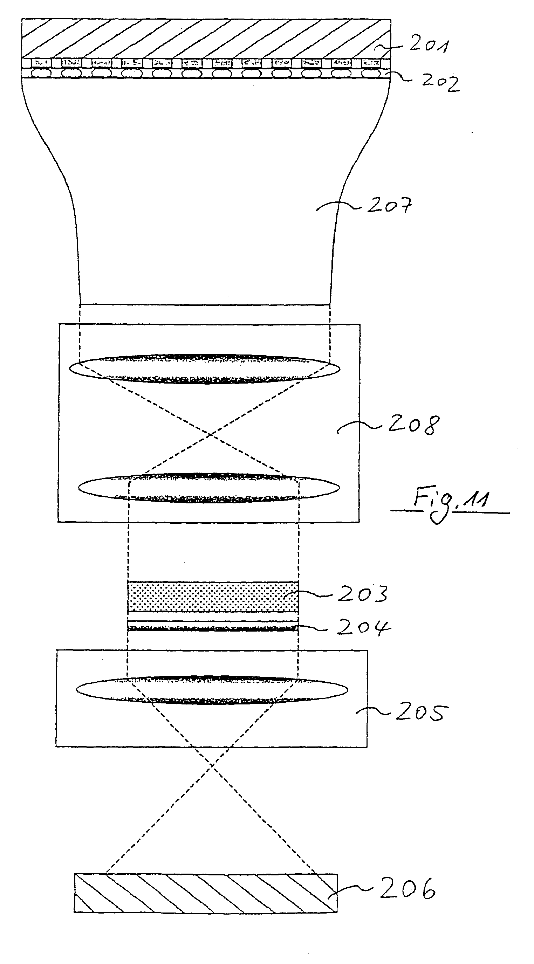

Die Figuren 9, 10 und 11 zeigen weitere mögliche Ausführungsformen einer erfindungsgemäßen Vorrichtung. All diesen Ausführungsformen ist gemeinsam, daß das von der Belichtungsmatrix 201 erzeugte Beleuchtungsmuster verkleinert auf dem Träger 203 abgebildet wird. Elemente in den Figuren 9, 10 und 11, die von ihrer Funktion her den Elementen in Fig. 8 entsprechen, sind mit jeweils korrespondierenden Bezugszeichen gekennzeichnet, so daß diesbezüglich auf die Beschreibung des Ausführungsbeispiels nach Fig. 8 verwiesen werden kann. Bei der Ausführungsform nach Fig. 9 erfolgt die Verkleinerung der optischen Abbildung durch Strahlführung in einem "fused fiber optic taper" 207, wohingegen im Ausführungsbeispiel nach Fig. 10 die Verkleinerung durch ein geeignetes abbildendes Linsensystem 208 realisiert wird. Hierbei können sowohl Makro- als auch Mikrolinsensysteme eingesetzt werden. Bei dem in Fig. 11 dargestellten Ausführungsbeispiel wird schließlich eine Kombination eines "fused fiber optic tapers" 207 und eines abbildenden Linsensystems 208 verwendet.

Zusammenfassend sei in bezug auf die Figuren 8-11 noch folgendes angemerkt. Sie zeigen eine Vorrichtung mit einer Lichtquellenmatrix, einer mikrooptischen Baukomponente sowie einer Verkleinerungsoptik zur lichtgesteuerten Induktion ortsaufgelöster chemischer bzw. biochemischer Synthesen in einem Reaktionsträger. Im Falle der Verwendung einer LED-Matrix als Lichtquellenmatrix hat es sich als zweckmäßig erwiesen, wenn nicht mehr als 75% der Lichtquellenmatrixfläche mit LED's bedeckt sind.

Es sei darauf hingewiesen, daß ein oder mehrere Spalte zwischen einzelnen Bauelementen der Vorrichtung mit einem optischen Fluid gefüllt sein können.

Bezugnehmend auf die in Figur 3 dargestellte Ausführungsform der Erfindung zeigt Figur 12 eine Anordnung umfassend einen transparenten Träger 105, z.B. eine kommerziell erhältliche Neubauer-Zell-Zell-Kammer, die mit farbigen Mikropartikeln 107 gefüllt worden ist. Bei Belichtung mit einer Lichtquelle 8, z.B. einer Kaltlichtquelle mit einer Faserauskopplung und Lochblende (802) können die farbigen Mikropartikel mit der CCD-Sensormatrix (16) detektiert und die Farbe durch Absorption bestimmt werden. Bei Markierung der Mikropartikel mit Fluoreszenzfarbstoffen kann eine Detektion durch den CCD-Sensor auf analoge Weise erfolgen.

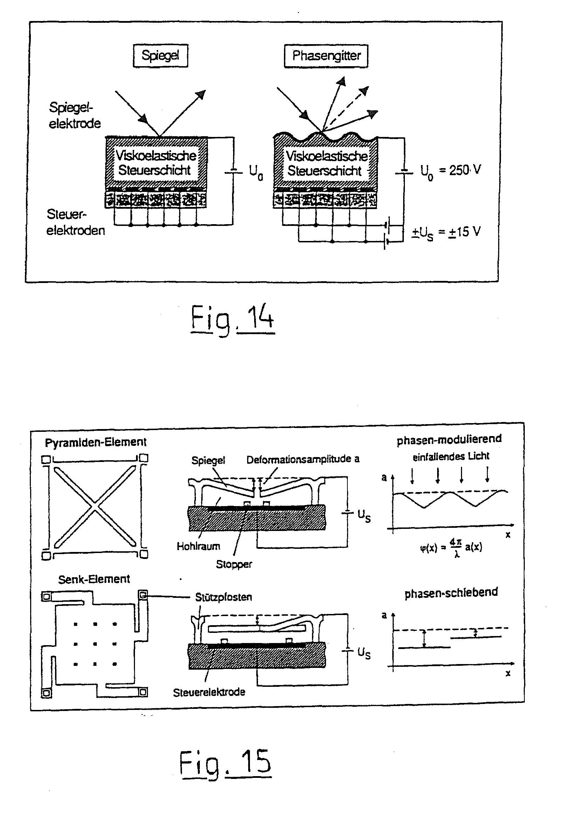

Die Fig. 14 und 15 sind den Datenblättern: "Lichtmodulatoren mit viskoelastischen Steuerschichten" und "Lichtmodulatoren mit mikromechanischen Spiegelarrays" des Fraunhofer-Institutes für Mikroelektronische Schaltungen und Systeme, IMS,1998, entnommen (dort jeweils Fig. 1).

Angaben aus dem Datenbalatt:
**"Lichtmodulatoren mit viskoelastischen Steuerschichten"**
des Fraunhofer-Instituts für Mikroelektronische Schaltungen und Systeme, IMS, D-01109 Dresden

### Merkmale

Viskoelastische Steuerschichten bilden eine Klasse von hochauflösenden Flächenlichtmodulatoren (SLM's) mit deformierbaren Spiegelanordnungen. Sie bestehen aus einem Array unabhängig adressierbarer Steuerelektroden auf einer unterliegenden aktiven CMOS-Ansteuermatrix, die mit einem viskoelastischen Silikongel beschichtet ist. Darauf wird eine dünne Aluminiumschicht aufgebracht, die eine geschlossene Spiegeloberfläche bildet und eine hohe Reflektivität im gesamten Bereich von IR bis DUV aufweist.

### Funktionsprinzip

Abb. 1 (wiedergegeben in Fig. 14 der vorliegenden Anmeldung) zeigt schematisch den Querschnitt einer viskoelastischen Steuerschicht. Zur Aktivierung wird eine Vorspannung zwischen Spiegel und Steuerelektroden gelegt, welche die Anordnung ganzflächig unter mechanischen Druck setzt. Die Oberfläche bleibt dabei zunächst glatt und wirkt für die Optik wie ein ebener Spiegel. Erst das Anlegen einer zusätzlichen Steuerspannung mit alternierender Polarität an benachbarte Steuerelektroden führt zu einer Deformation aufgrund der sich ändernden elektrischen Feldkräfte. Durch Wechsel der Polarität entweder in einer oder in beiden Raumrichtungen lassen sich dabei jeweils 1D- oder 2D-sinusförmige Deformationsprofile erzeugen.

Abb. 2 (des IMS-Datenblattes) zeigt dazu die an einem Steg-Spalt-Muster gemessenen Oberflächenprofile. Optisch stellen diese Deformationsprofile Phasengitter dar, deren Gitterperiode durch den Steuerelektrodenabstand definiert wird. Das einfallende Licht erfährt dabei eine Phasenmodulation entsprechend der durch die Spiegeldeformation gegebenen optischen Gangunterschiede. Durch geeignete Wahl der Deformationsamplitude kann hier nahezu das gesamte Licht in höhere Beugungsordnungen gebeugt werden, wohingegen das Licht von nicht-adressierten ebenen Pixeln allein in die nullte Ordnung fällt.

In Verbindung mit einem geeigneten optischen System kann dann erreicht werden, dass nur das Licht von nicht-adressierten Bereichen durchgelassen und als sichtbares Intensitätsmuster in die Bildebene projiziert wird.

Viskoelastische Steuerschichten eignen sich daher gut zur Erzeugung von Phasenmustern für optische Abbildungsanwendungen. Mit dieser Technologie wurde ein binär arbeitender SLM-Prototyp mit aktiver Matrix aus 1024 x 2048 Pixeln und 20 x 20 µm² Pixelgröße entwickelt, wobei ein spezieller Hochvolt-CMOS-Prozess zur Ermöglichung von Steuerspannungen bis zu ± 15 V eingesetzt wurde.

Abb. 3 (des IMS-Datenblattes) zeigt dazu das schematische Layout der aktiven Steuermatrix. Die Funktion dieses Prototyps wurde erfolgreich in einer Applikation zur schnellen Laserdirektbelichtung von sub-µ-Strukturen demonstriert. Er diente dabei zur Erzeugung von Phasenmustern aus den CAD-Layout-Daten von IC-Maskenlayern, die dann mit Hilfe des optischen Systems in ein Intensitätsbild zur Fotolackstrukturierung umgesetzt wurden.

Gegenwärtig befinden sich Lichtmodulatoren in der Entwicklung, die einen 4 Bit-Analogbetrieb und eine Staffelung der Bildfeldgröße in Schritten von 256 Pixeln je Richtung erlauben.

### Anwendungen

Lichtmodulatoren mit viskoelastischen Steuerschichten eröffnen viele neue Anwendungsmöglichkeiten:
Display-Technologie:
   - Video- und Daten-Projektion
   - Head-up-Displays

Informationstechnologie:
- Optische Bild- und Datenverarbeitung
- optische Speicher

Fertigungstechnologie:
- Maskenloses Direktschreiben
- Laserablation und -fertigung

### Technische Parameter

| | |
|---|---|
| Pixelgröße | > 16x16 µm² |
| Pixelzahl | 256 x 256 ... 1024 x 2048 |
| Profil | 1D, 2D sinusförmig |
| Modulation | phasenmodulierend |
| Betrieb | binär oder 4 Bit analog |
| Deformationsamplitude | 0 ..... 150 nm |
| Steuerkurve | nahezu linear |
| Einstellzeit | < 2 ms |
| Dateneingänge | 16, 32, 48, 64 Kanäle, 4 Bit, 5 V |
| Bildrate | 100 Hz .... 500 Hz |
| opt. Füllgrad | 100 % |
| Reftektivität | > 90 % (IR .... DUV) |
| Aufbautechnik | keramisches PGA-Gehäuse |

### User Evaluation Kit

Um die Möglichkeit anzubieten, alle grundlegenden SLM-Funktionen in einem anwenderspezifischen Umfeld zu testen, wurde ein User Evaluation Kit mit allen Komponenten für eine nutzerspezifische Bildprogrammierung der SLM's entwickelt.

### SLM

| | |
|---|---|
| Pixelgröße | 16 x 16, 20 x 20, 24 x 24 µm² |
| Pixelzahl | 256 (160) x 256 |
| Pixeldesign | kundenspezifisch |
| Betrieb | 4 Bit analog |

### SLM-board

| | |
|---|---|
| RAM | Speicherung von 2 Bildern |
| Bildrate | 1 Hz (PC zu RAM) 500 Hz (RAM zu SLM) |
| I/O-Signale | Matrix Trigger, Matrix Ready |

### Datentransfer

- über Kabelverbindung und digitaler I/O-Interface-Karte für ISA-Slot am PC

### Software

- Konversion der Nutzerbilddaten von Bitmap in das SLM-Datenformat
- Kontrollfunktionen zum Datentransfer
- Einstellung der Steuerspannungspegel für 4 Bit Graustufen

### Anforderungen

- Windows-kompatibler PC
- Bildmustererzeugung im Bitmap-Datenformat, z.B. mit Paintbrush

Angaben aus dem Datenblatt:
**"Lichtmodulatoren mit mikromechanischen Spiegelarrays"**
des Fraunhofer-Institutes für Mikroelektronische Schaltungen und Systeme, IMS, D-01109 Dresden

### Merkmale

Mikromechanische Spiegelarrays bilden eine Klasse von hochauflösenden Flächenlichtmodulatoren (SLM's) mit deformierbaren Spiegelanordnungen. Sie bestehen aus einem Array unabhängig adressierbarer Mikrospiegel, die mit den Methoden der Oberflächen-Mikromechanik in einem komplett CMOS-kompatiblen Prozess auf einer unterliegenden aktiven Matrix-Steuerschaltung hergestellt werden. Der Prozess benötigt lediglich drei zusätzliche Masken und erlaubt damit eine einfache Anpassung der lichtmodulierenden Eigenschaften an die verschiedensten anwendungsspezifischen Erfordernisse durch bloße Änderung der Spiegelarchitektur.

### Funktionsprinzip

Die Mikrospiegel werden mit einer Opferschicht-Technik hergestellt, so dass freitragende Spiegelelemente über einem Hohlraum mit unterliegender Steuerelektrode entstehen.

Spiegel und Stützpfosten bestehen dabei gleichermaßen aus Aluminium, um eine hohe Reflektivität über einem breiten Spektralbereich von IR bis DUV zu gewährleisten.

Die Aktivierung erfolgt durch Anlegen einer Steuerspannung zwischen Spiegel und Steuerelektrode, so dass die Spiegel infolge der elektrostatischen Kraftwirkung in den Hohlraum hinein deformiert werden. Die damit verbundenen optischen Gangunterschiede führen beim einfallenden Licht zu einer entsprechenden Phasenmodulation. Das Deformationsprofil und damit die lichtmodulierenden Eigenschaften hängen dabei stark von der jeweiligen Spiegelarchitektur ab. Hier lassen sich die drei grundlegenden Fälle phasenmodulierend, phasenschiebend und lichtumlenkend unterscheiden.

Aus der Vielzahl der möglichen Pixelarchitekturen wurden die beiden Strukturen aus Abb. 1 (wiedergegeben in Fig. 15 der vorliegenden Anmeldung) näher untersucht.

Bei der ersten Variante werden durch elektrostatische Deformation von vier identischen Spiegelsegmenten optische Phasengitter erzeugt, in denen ein Pixel jeweils eine Gitterperiode mit inversem pyramidenförmigen Phasenprofil definiert. Diese Variante eignet sich gut zur Generierung von Phasenmustern für optische Abbildungsanwendungen.

Die zweite Variante besteht aus einer von vier Armen gehaltenen Spiegelplatte, die bei elektrischer Ansteuerung eine ebene, kolbenartige Senkbewegung liefert und damit eine pixelweise Einstellung der Phase des einfallenden Lichts erlaubt. Diese Variante eignet sich gut zur Phasenfrontkorrektur in adaptiven Optiken.

Derartige Mikrospiegel wurden bereits auf passiven Matrizen zur Untersuchung der elektromechanischen Eigenschaften aufgebaut (Abb. 2, 3 des IMS-Datenblattes). Die gemessenen Kurven in Abb. 4 (des IMS-Datenblattes) zeigen dazu das typische Deformationsverhalten. Im Analogbereich steigt die Deformation nahezu quadratisch mit der Steuerspannung an. Oberhalb des sogenannten Pull-in-Points schalten die Spiegel jedoch aufgrund der Mitkopplung durch das elektrische Feld spontan in den voll ausgelenkten Zustand, so dass hier nur noch ein binärer Betrieb möglich ist. Um die Spiegel schließlich wieder in einen Gleichgewichtszustand zwischen mechanischen und elektrischen Kräften zurückzusetzen, ist eine entsprechende Reduzierung der Steuerspannung erforderlich.

### Anwendungen

Lichtmodulatoren mit mikromechanischen Spiegeln eröffnen eine Vielzahl von Anwendungsmöglichkeiten:
Displaytechnologie:
   - Video- und Daten-Projektion
   - Head-up-Displays

Informationstechnologie:
- Optische Bild- und Datenverarbeitung
- Optische Speicher

Phasenfrontkorrektur adaptiven Optiken

Fertigungstechnologle:
- Maskenloses Direktschreiben
- Laserablation und -fertigung

Medizintechnik:
- Laser-Scanning-Tomographie
- Laser-Chirurgie
- Endoskopische Head-up-Displays

### Technische Parameter

| | |
|---|---|
| Pixelgröße | > 16x16 µm² |
| Pixelzahl | 256 x 256 ... 1024 x 2048 |
| Pixeldesign | kundenspezifisch, Pyramiden-, Senk-, Torsionselemente, ... |
| Profil | pyramiden-, rechteckförmig, Sägezahn, .... |
| Modulation | phasenmodulierend bzw. -schiebend, umlenkend |
| Betrieb | binär oder 4 Bit analog |
| Deformationsamplitude | 0 .... 1,2 µm (analog) bis zu 5,0 µm (binär) |
| Steuerkurve | nicht-linear |
| Einstellzeit | 10 µs (typ.) |
| Bildrate | 100 Hz .... 1 kHz |
| opt. Füllgrad | 80 ..... 90% |
| Reflektivität | > 90% (IR .... DUV) |

### User Evaluation Kit

Um die Möglichkeit anzubieten, alle grundlegenden SLM-Funktionen in einem anwenderspezifischen Umfeld zu testen, wurde ein User Evaluation Kit mit allen Komponenten für eine nutzerspezifische Bildprogrammierung der SLM's entwickelt.

### SLM

| | |
|---|---|
| Pixelgröße | 16 x 16, 20 x 20, 24 x 24 µm² |
| Pixelzahl | 256 (160) x 256 |
| Pixeldesign | kundenspezifisch |
| Betrieb | 4 Bit analog |

### SLM-board

| | |
|---|---|
| RAM | Speicherung von 4 Bildern |
| Bildrate | 1 Hz (PC zu RAM) 500 Hz (RAM zu SLM) |
| I/O-Signale | Matrix Trigger, Matrix Ready |

### Datentransfer

- über Kabelverbindung und digitaler I/O-Interface-Karte für ISA-Slot am PC

### Software

- Konversion der Nutzerbilddaten von Bitmap in das SLM-Datenformat
- Kontrollfunktionen zum Datentransfer
- Einstellung der Steuerspannungspegel für 4 Bit Graustufen

### Anforderungen

- Windows-kompatibler PC
- Bildmustererzeugung im Bitmap-Datenformat, z.B. mit Paintbrush

## Patentansprüche

1. Verfahren zur Herstellung eines mit biologisch oder chemisch funktionellen Materialien beschichteten Trägers (BioChip) umfassend die Schritte:
(a) Bereitstellen eines Trägers mit einer Oberfläche, die photoaktivierbare Gruppen aufweist,
(b) automatisches Erkennen und/oder Kalibrieren des Trägers mittels einer Lichtsensormatrix und einer dieser nachgeschalteten Auswerteeinheit,
(c) Aktivieren der photoaktivierbaren Gruppen auf mindestens einem vorbestimmten Bereich der Trägeroberfläche durch ortsspezifische Belichtung des Trägers mit einer
Belichtungsmatrix, die zur Erzeugung eines wahlweise einstellbaren Belichtungsmusters steuerbar ist,
(d) ortsspezifisches Binden von biologisch oder chemisch funktionellen Materialien oder Bausteinen für solche Materialien auf mindestens einem der vorbestimmten Bereiche und
(e) gegebenenfalls Wiederholen der Aktivierungs- und Bindeschritte auf gleichen oder/und unterschiedlichen vorbestimmten Bereichen.

2. Verfahren zur Herstellung eines mit biologisch oder chemisch funktionellen Materialien beschichteten Trägers (BioChip) nach Anspruch 1, ferner umfassend den Schritt der Systemeichung und/oder Systemkalibrierung unter Verwendung der Lichtsensormatrix.

3. Verfahren nach einem der Ansprüche 1 oder 2, **dadurch gekennzeichnet, dass** die biologisch oder chemisch funktionellen Materialien ausgewählt werden aus Nukleinsäuren und Nukleinsäurebausteinen, insbesondere Nukleotiden und Oligonukleotiden, Nukleinsäureanaloga wie PNA und Bausteinen davon, Peptiden und Proteinen und Bausteinen davon, insbesondere Aminosäuren, Sacchariden, Zellen, subzellulären Präparationen, wie Zellorganellen oder Membranpräparationen, viralen Partikeln, Zellaggregaten, Allergenen, Pathogenen, pharmakologischen Wirkstoffen und diagnostischen Reagenzien.

4. Verfahren nach einem der Ansprüche 1 bis 3, **dadurch gekennzeichnet, dass** die biologisch oder chemisch funktionellen Materialien durch mehrstufigen Aufbau aus Monomer- oder/und Oligomerbausteinen auf dem Träger synthetisiert werden.

5. Verfahren nach einem der Ansprüche 1 bis 4, **dadurch gekennzeichnet, dass** die Aktivierung von vorbestimmten Bereichen eine Schutzgruppenabspaltung vom Träger selbst oder darauf gebundenen Materialien oder Bausteinen davon umfasst.

6. Vorrichtung zur Herstellung eines mit biologisch oder chemisch funktionellen Materialien beschichteten Trägers (BioChip), umfassend
eine Belichtungsmatrix (10), die zur Erzeugung eines wahlweise einstellbaren Belichtungsmusters mittels einer programmierbaren Steuereinrichtung (12) steuerbar ist,
einen die Belichtungsmatrix (10) tragenden Rahmen mit einer Trägerhalterung zur Aufnahme und Positionierung eines betreffenden Trägers (4) relativ zur Belichtungsmatrix, so dass von der Belichtungsmatrix (10) erzeugte Belichtungsmuster auf die Oberfläche des Trägers (4) projizierbar sind, um diesen zur Aktivierung photoaktivierbarer Gruppen auf mindestens einem vorbestimmten Bereich seiner Oberfläche ortsspezifisch zu belichten,
**dadurch gekennzeichnet, dass** die Belichtungsmatrix als selbstemittierende Belichtungsmatrix, insbesondere als Diodenarray oder/und Laserarray, ausgebildet ist.

7. Vorrichtung nach Anspruch 6, wobei sie eine optische Lichtdetektionseinrichtung (6) mit einer Lichtsensormatrix (16) zur Beobachtung des Trägers (4) aufweist.

8. Vorrichtung nach Anspruch 7, wobei die Lichtsensormatrix (16) dazu eingerichtet ist, mittels einer ihr nachgeschalteten Auswerteeinheit den Träger (4) automatisch zu erkennen und/oder zu kalibrieren.

9. Vorrichtung nach Anspruch 7 oder 8, wobei die Vorrichtung dazu eingerichtet ist, mittels der Lichtdetektionseinrichtung (6) eine Systemeichung oder Systemkalibrierung durchzuführen.

10. Vorrichtung nach Anspruch 7 , 8 oder 9, wobei die Lichtsensormatrix (16) dazu eingerichtet ist, zur Durchführung einer insbesondere permanenten Prozesskontrolle verwendet zu werden.

11. Vorrichtung nach einem der Ansprüche 7 bis 10, wobei die Lichtsensormatrix (16) als CCD-Sensormatrix ausgebildet ist.

12. Vorrichtung nach einem der vorhergehenden Ansprüche 7 bis 11, wobei die Belichtungsmatrix (10), der Träger (4) und die Lichtsensormatrix (16) eine Durchlichtanordnung bilden.

13. Vorrichtung nach einem der Ansprüche 7 bis 12, wobei die Belichtungsmatrix (10), der Träger (4) und die Lichtsensormatrix (16) eine Auflichtanordnung bilden.

14. Vorrichtung nach einem der Ansprüche 6 bis 13, ferner umfassend Elemente zur Verkleinerung der optischen Abbildung auf den Reaktionsträger.

15. Verwendung der Vorrichtung nach einem der Ansprüche 6 bis 14 zur Herstellung eines mit biologisch oder chemisch funktionellen Materialien beschichteten Trägers (BioChip).
